# EUROPEAN PATENT SPECIFICATION

(11) **EP 3 160 494 B1**
(45) Date of publication and mention of the grant of the patent: **20.11.2019**
(21) Application number: 15812408.1
(22) Date of filing: 25.06.2015
(51) Int. Cl.: A61K 38/47, C12P 21/00, C12N 9/24

(54) **GENERATION OF MANNOSE-6-PHOSPHATE CONTAINING RECOMBINANT ALPHA-N-ACETYL GLUCOSAMINIDASE**
ERZEUGUNG VON MANNOSE-6-PHOSPHAT MIT REKOMBINANTER ALPHA-N-ACETYL-GLUCOSAMINIDASE
PRODUCTION D'ALPHA-N-ACÉTYL-GLUCOSAMINIDASE RECOMBINANTE CONTENANT DU MANNOSE-6-PHOSPHATE

(30) Priority: 25.06.2014 US 201462017142 P
(43) Date of publication of application: 03.05.2017
(73) Proprietor: Shire Human Genetic Therapies, Inc., Lexington, MA 02421 (US)
(72) Inventor: ZHANG, Bohong, Lexington, Massachusetts 02421 (US); CONCINO, Michael F., Lexington, Massachusetts 02421 (US); NORTON, Angela W., Lexington, Massachusetts 02421 (US); MEIYAPPAN, Muthuraman, Lexington, MA 02421 (US); LUNDBERG, Dianna, Lexington, MA 02421 (US)
(74) Representative: Carpmaels & Ransford LLP
(86) International application number: PCT/US2015/037756
(87) International publication number: WO 2015/200675

(56) References cited:
- WO-A2-03/057710
- WO-A2-2009/131698
- WO-A2-2009/131698
- WO-A2-2011/163652
- WO-A2-2012/012742
- WO-A2-2012/012742
- US-A1- 2003 119 088
- US-A1- 2003 119 088
- US-A1- 2013 095 092
- US-A1- 2013 095 092
- US-B2- 7 001 994
- US-B2- 7 001 994
- YOGALINGAM G ET AL: "Mucopolysaccharidosis type IIIB: characterisation and expression of wild-type and mutant recombinant alpha-N-acetylglucosaminidase and relationship with Sanfilippo phenotype in an attenuated patient", BIOCHIMICA ET BIOPHYSICA ACTA. MOLECULAR BASIS OF DISEASE, AMSTERDAM, NL, vol. 1502, no. 3, 15 November 2000 (2000-11-15), pages 415-425, XP027370235, ISSN: 0925-4439 [retrieved on 2000-11-15]
- ROSSOMANDO ANTHONY ET AL: "SBC-103, a recombinant human alpha-N-acetylglucosaminidase, demonstrates mannose-6-phosphate receptor dependent transport in an in vitro blood-brain barrier model", MOLECULAR GENETICS AND METABOLISM, vol. 111, no. 2, 27 January 2014 (2014-01-27), XP028821435, ISSN: 1096-7192, DOI: 10.1016/J.YMGME.2013.12.219
- ZHAO K-W ET AL: "Purification and Characterization of Recombinant Human @a-N-Acetylglucosaminidase Secreted by Chinese Hamster Ovary Cells", PROTEIN EXPRESSION AND PURIFICAT, ACADEMIC PRESS, SAN DIEGO, CA, vol. 19, no. 1, 1 June 2000 (2000-06-01), pages 202-211, XP004435533, ISSN: 1046-5928, DOI: 10.1006/PREP.2000.1230
- WEBER BIRGIT ET AL: "Expression and characterization of human recombinant and alpha-N-actylglucosaminidase", PROTEIN EXPRESSION AND PURIFICATION, ACADEMIC PRESS, SAN DIEGO, CA, vol. 21, no. 2, 1 March 2001 (2001-03-01), pages 251-259, XP002740102, ISSN: 1046-5928, DOI: 10.1006/PREP.2000.1361
- YOGALINGAM, G ET AL.: 'Mucopolysaccharidosis Type IIIB: Characterisation And Expression Of Wild-Type And Mutant Recombinant K-N-Acetylglucosaminidase And Relationship With Sanfilippo Phenotype In An Attenuated Patient.' BIOCHIM BIOPHYS ACTA vol. 1502, no. 3, 15 November 2000, ISSN 0925-4439 pages 415 - 425, XP027370235

## Description

### BACKGROUND

More than forty lysosomal storage diseases are caused, directly or indirectly, by the absence or deficiency of one or more lysosomal enzymes. Sanfilippo syndrome, or mucopolysaccharidosis III (MPS III), is one such disease. It is a rare genetic disorder characterized by the deficiency of enzymes involved in the degradation of glycosaminoglycans (GAG).

Four distinct forms of MPS III, designated MPS IIIA, B, C, and D, have been identified. Each of these forms is caused by a deficiency in a different lysosomal enzyme. Mucopolysaccharidosis type IIIB (MPS IIIB; Sanfilippo B disease) is an autosomal recessive disorder that is characterized by a deficiency of the enzyme alpha-N-acetyl-glucosaminidase (Naglu), resulting in the accumulation of heparan sulfate in lysosomes of neurons and glial cells, with lesser accumulation outside the brain. Methods for treating MPS IIIB are disclosed in WO 2011/163652 and WO 2012/012742.

Enzyme replacement therapy (ERT) has been used for the treatment of various lyosomal storage diseases to deliver enzymes to patients in whom that particular enzyme is deficient or absent. Normally, lysosomal enzymes are synthesized in the cytosol and traverse the endoplasmic reticulum (ER) where they are glycosylated with N-linked, high mannose type carbohydrates. In the Golgi apparatus, the high mannose carbohydrates on glycoproteins (e.g., lysosomal enzymes) are then modified by the addition of mannose-6-phosphate (M6P) groups. This modification targets the proteins to the lysosome via binding to the cation-independent mannose-6-phosphate receptor (CI-M6PR). Efficient targeting of enzymes to the lysosome is the key to successful enzyme replacement therapy.

However, replacement enzymes are typically produced *in vitro,* using a cell culture system, and often do not have the level and type of glycosylation necessary for efficient lysosomal targeting. In the case of some lysosomal enzymes, such as recombinantly produced Naglu protein, there is a dramatic reduction of glycosylation and, in particular, M6P phosphorylation. This presents a formidable obstacle to the treatment of lysosomal storage diseases, such as Sanfilippo B syndrome, with replacement enzymes.

Therefore, there remains a need to develop alternative methods for lysosomal targeting to ensure effective enzyme replacement therapy.

A method for producing high mannose glycoproteins in complex carbohydrate deficient cells is disclosed in WO 2003/057710. A method for producing recombinant Naglu is disclosed in US 2013/0095092. US 2003/0119088 describes a soluble GlcNAc phosphotransferase, as well as methods of making the same and a method of phoshorylating with the same.

### SUMMARY

The present invention provides an improved method for producing mannose-6-phosphate (M6P)-containing recombinant alpha-N-acetyl-glucosaminidase (Naglu) as defined in the claims, which is suitable for effective enzyme replacement therapy of Sanfilippo syndrome type B. The present invention addresses, among other things, a lack of M6P glycans that has been observed in certain recombinant Naglu. A lack of M6P glycans in recombinant Naglu has been observed using, e.g., CHO, HEK293, and human skin fibroblast cells, while endogenous Naglu from these cell types does include M6P glycans. Lack of M6P in recombinant Naglu presents a challenge in Enzyme Replacement Therapy for, e.g., Sanfilippo B syndrome. This can be because at least one route by which cells internalize lysosomal enzymes can be facilitated by receptors that bind M6P, such as cation-dependent M6P receptor. Accordingly, certain embodiments of the present invention are based, in part, on the finding that recombinant Naglu that does not bind to M6P receptor may not be internalized by cells. The present invention includes, among other things, insight as to why recombinant Naglu is not phosphorylated and the development of methods and compositions to allow for the addition of M6P to recombinant Naglu.

In one aspect, the present invention provides a method of producing mannose-6-phosphate (M6P)-containing recombinant alpha-N-acetyl-glucosaminidase (Naglu), comprising the steps of providing a high mannose containing Naglu protein which has been generated from mammalian or non-mammalian cells using recombinant DNA technology; and contacting the high mannose containing recombinant Naglu protein with N-acetylglucosamine-1-phosphotransferase (GNPT) under conditions that permit phosphorylation of one or more mannose residues on the recombinant Naglu protein, wherein conditions that permit phosphorylation comprise a greater than 1:1 GNPT to Naglu molar ratio such that GNPT is in excess of Naglu, thereby generating M6P-containing recombinant Naglu.

In some embodiments, the high mannose containing recombinant Naglu protein comprises substantially Man9(GlcNAc)2, Man8(GlcNAc)2, Man7(GlcNAc)2, Man6(GlcNAc)2, and/or Man5(GlcNAc)2 N-linked oligosaccharides. In some embodiments, the high mannose containing recombinant Naglu protein comprises substantially Man9(GlcNAc)2 N-linked oligosaccharides.

In some embodiments, the high mannose containing recombinant Naglu protein is produced from cells cultured in the presence of an alpha-mannosidase I inhibitor. In some embodiments, a suitable alpha-mannosidase I inhibitor is selected from the group consisting of deoxymannojirimycin, kifunensine, D-Mannonolactam amidrazone, N-butyl-dexoymannojirimycin, and combinations thereof. In some embodiments, a suitable alpha-mannosidase I inhibitor is kifunensine.

In some embodiments, suitable cells are mammalian cells. In some embodiments, suitable cells are human cells. In some embodiments, suitable cells are CHO cells.

In some embodiments, a method according to the present invention further includes a step of purifying the high mannose containing recombinant Naglu protein from the cells.

In some embodiments, the step of contacting the high mannose containing recombinant Naglu protein with the GNPT is carried out in vitro. In some embodiments, the step of contacting the high mannose containing recombinant Naglu protein with the GNPT is carried out in a reaction vessel.

The conditions that permit phosphorylation include providing the GNPT in an amount greater than the recombinant Naglu protein. In some embodiments, the conditions that permit phosphorylation include incubating the high mannose containing recombinant Naglu protein with the GNPT in the presence of UDP-GlcNac. In some embodiments, the conditions that permit phosphorylation include incubating the high mannose containing recombinant Naglu protein with the GNPT at about 20°C.

In some embodiments, the method further includes a step of incubating the M6P-containing recombinant Naglu with N-acetyl-glucosamine 1-phosphodiester α-N-acetyl-glucosaminidase.

In some embodiments, the recombinant Naglu protein comprises an amino acid sequence of SEQ ID NO:1. In some embodiments, the recombinant Naglu protein is not fused or conjugated to a peptide.

In another aspect, the present disclosure provides a mannose-6-phosphate (M6P)-containing recombinant alpha-N-acetyl-glucosaminidase (Naglu) protein produced by a method described herein.

In yet another aspect, the present disclosure provides a composition comprising recombinant alpha-N-acetyl-glucosaminidase (Naglu) protein characterized by mannose-6-phosphate (M6P) containing glycans. In some embodiments, the M6P containing glycans comprise capped-M6P, 1M6P and/or 2M6P containing glycans.

In some embodiments, the recombinant Naglu protein produced by the method of the present invention is further characterized by high mannose containing glycans and/or sialic acid containing glycans. In some embodiments, the sialic acid containing glycans comprise one, two, three, and/or four sialic acid containing glycans.

In a further aspect, the present disclosure provides a composition comprising recombinant alpha-N-acetyl-glucosaminidase (Naglu) protein characterized by substantially high mannose containing glycans. In some embodiments, the high mannose containing glycans contain Man9(GlcNAc)2, Man8(GlcNAc)2, Man7(GlcNAc)2, Man6(GlcNAc)2, and/or Man5(GlcNAc)2 N-linked oligosaccharides. In some embodiments, the high mannose containing glycans comprises substantially Man9(GlcNAc)2 N-linked oligosaccharides.

In some embodiments, the recombinant Naglu protein is produced from mammalian cells. In some embodiments, the mammalian cells are human cells. In some embodiments, the mammalian cells are CHO cells.

In some embodiments, the recombinant Naglu protein provided herein comprises an amino acid sequence of SEQ ID NO:1. In some embodiments, the recombinant Naglu protein provided herein is not fused or conjugated to a peptide.

A composition produced according to the present invention can be a pharmaceutical composition further comprising a pharmaceutically acceptable carrier.

Sanfilippo Syndrome Type B (MPS IIIB) may be treated by administering to a subject in need of treatment a therapeutically effective amount of a recombinant M6P-containing Naglu protein or pharmaceutical composition containing the same.

Other features, objects, and advantages of the present invention are apparent in the detailed description, drawings and claims that follow. It should be understood, however, that the detailed description and the drawings, while indicating embodiments of the present invention, are given by way of illustration only, not limitation.

### BRIEF DESCRIPTION OF THE FIGURES

FIG. 1 shows the hydrolysis of UDP-GlcNAc by rhNaglu. FIG. 1A shows an overlay of chromatograms derived from samples in which UDP-GlcNAc was hydrolyzed with increasing concentrations of rhNaglu. Inserts show the peaks for UDP-GlcNAc and GlcNAc at 40.9 and 10.7 minutes, respectively.
FIG. 1B shows the Area Under the Curve (AUC) of the UDP-GlcNAc-derived peaks of FIG. 1A as a function of Naglu concentrations used to hydrolyze UDP-GlcNAc.
FIG. 1C shows the Area Under the Curve (AUC) of the GlcNAc-derived peaks of FIG. 1A as a function of Naglu concentrations used to hydrolyze UDP-GlcNAc.
FIG. 1D shows an overlay of chromatograms derived from samples with increasing amounts of UDP-GlcNAc. This overlay demonstrates the elution time of UDP-GlcNAc at 40.9 minutes.
FIG. 1E shows an overlay of chromatograms derived from samples with increasing amounts of GlcNAc. This overlay demonstrates the elution time of GlcNAc at 10.7 minutes.
FIG. 2 shows rhNaglu did not hydrolyze UDP-GlcNAc's epimer, UDP-GalNAc. FIG. 2A shows an overlay of chromatograms derived from samples in which UDP-GalNAc was treated with increasing concentrations of rhNaglu. FIG. 2B shows the Area Under the Curve (AUC) of the GalNAc-derived peaks of FIG. 2A as a function of Naglu concentrations used to treat UDP-GalNAc.
FIG. 3 shows hydrolysis of UDP-GlcNAc by Naglu was dependent on the initial concentration of UDP-GlcNAc. FIG. 3A shows the AUC of UDP-GlcNAc-derived peaks as a function of increasing concentration of initial UDP-GlcNAc input.
FIG. 3B shows the AUC of GlcNAc-derived peaks as a function of increasing concentration of UDP-GlcNAc input.
FIG. 4 shows the effect of UDP-GlcNAc, GlcNAc, UDP-GalNAc and Glucosamine on the activity of Naglu.
FIG. 5 shows HI-UHPLC column separation of 2-AB labeled glycans released from rhNaglu. N-linked glycans present on rhNaglu were released with PNGase F, labeled with 2-AB and separated using HI-UHPLC.

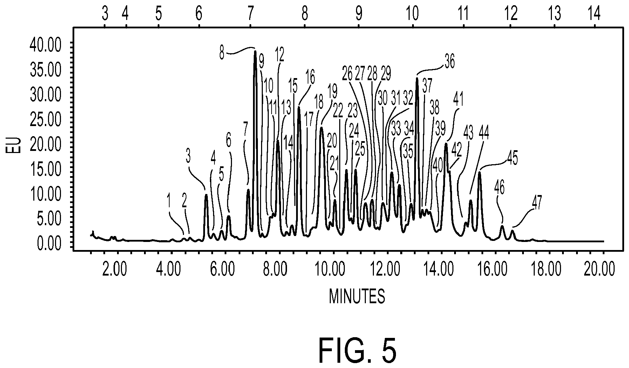

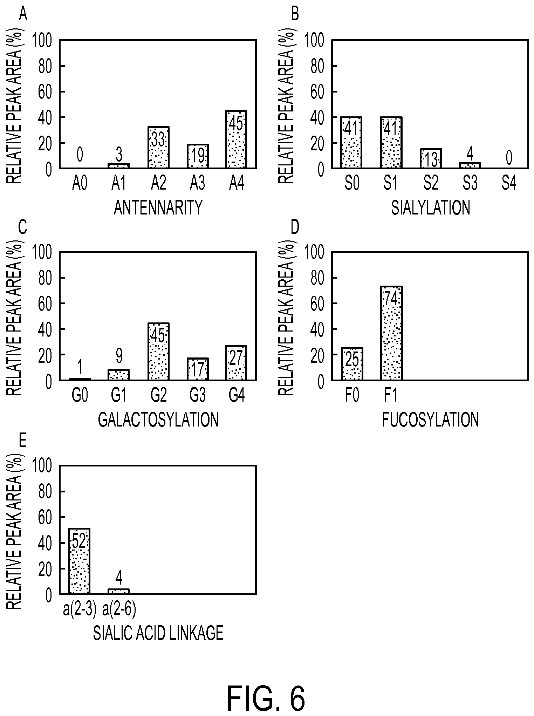

FIG. 6 shows a summary of major characteristics of glycan structures on rhNaglu. The AUC values of the glycan-derived peaks of FIG. 5 were used to calculate the relative abundance of different types of glycan on rhNaglu. FIG. 6A shows the extent of antennarity of the glycans which was determined by adding peak areas of structures containing either no, one, two, three or four GlcNAcs, denoted as A0, A1, A2, A3 or A4, respectively. FIG. 6B shows the extent of terminal sialylation which was determined by adding peak areas of structures containing no, one, two, three, or four terminal sialic acids (S0, S1, S2, S3 or S4, respectively). FIG. 6C shows the extent of the addition of galactose onto GlcNAcs which was determined by adding peak areas of such structures having no, one, two, three or four galactose units (G0, G1, G2, G3 or G4, respectively). FIG. 6D shows the extent of core fucosylation which was determined by adding peak areas of structures either not having or having core fucose (F0 or F1, respectively). Figure 6E shows the abundance of different sialic acid linkages that was determined by adding peak areas of structures carrying these modifications.
FIG. 7 shows Coomassie Blue staining of rhNaglu and Naglu-Kif, after treatment with Endo H and PNGase F and separation by SDS-PAGE.
FIG. 8 shows Naglu-Kif modified by GNPT in vitro resulted in increased binding to CI-M6PR.
FIG. 9 shows the quantification of M6P monosaccharide derived from GNPT-modified Naglu-Kif by PAD-HPLC at different concentrations of sGNPT. FIG. 9A-C show M6P quantification of reactions where 140, 70 and 35 µg of GNPT were used, respectively.
FIG. 10 shows a glycan profile of Naglu-Kif after phosphorylation by sGNPT.
FIG. 11 shows alkaline phosphatase, alpha-mannosidase and neuroaminidase treatment of the glycans to further confirm the identity of the glycan peaks.

### DEFINITIONS

In order for the present invention to be more readily understood, certain terms are first defined below. Additional definitions for the following terms and other terms are set forth throughout the specification.

*Alpha-N-acetyl-glucosaminidase*: As used herein, the term "alpha-N-acetyl-glucosaminidase" refers to enzymes capable of hydrolyzing terminal non-reducing N-acetyl-D-glucosamine residues in N-acetyl-alpha-D-glucosaminides. Alpha-N-acetyl-glucosaminidase is also known as alpha-acety-glucosaminidase, N-acetyl-alpha-D-glucosaminidase, N-acetyl-alpha-glucosaminidase and alpha-D-2-acetamido-2-deoxyglucosidase.

*Approximately or about:* As used herein, the term "approximately" or "about," as applied to one or more values of interest, refers to a value that is similar to a stated reference value. The term "approximately" or "about" can refer to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Amino acid*: As used herein, term "amino acid," in its broadest sense, refers to any compound and/or substance that can be incorporated into a polypeptide chain. An amino acid can have the general structure H₂N-C(H)(R)-COOH. An amino acid can be a naturally occurring amino acid. An amino acid can be a synthetic amino acid; an amino acid can be a d-amino acid; an amino acid can be an 1-amino acid. "Standard amino acid" refers to any of the twenty standard 1-amino acids commonly found in naturally occurring peptides. "Nonstandard amino acid" refers to any amino acid, other than the standard amino acids, regardless of whether it is prepared synthetically or obtained from a natural source. As used herein, "synthetic amino acid" encompasses chemically modified amino acids, including but not limited to salts, amino acid derivatives (such as amides), and/or substitutions. Amino acids, including carboxy- and/or amino-terminal amino acids in peptides, can be modified by methylation, amidation, acetylation, protecting groups, and/or substitution with other chemical groups that can change the peptide's circulating half-life without adversely affecting their activity. Amino acids may participate in a disulfide bond. Amino acids may comprise one or posttranslational modifications, such as association with one or more chemical entities (*e*.*g*., methyl groups, acetate groups, acetyl groups, phosphate groups, formyl moieties, isoprenoid groups, sulfate groups, polyethylene glycol moieties, lipid moieties, carbohydrate moieties, biotin moieties, *etc*.)*.* The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Biologically active*: As used herein, the phrase "biologically active" (or biological activity) refers to a characteristic of any agent that has activity in a biological system, and particularly in an organism. For instance, an agent that, when administered to an organism, has a biological effect on that organism, is considered to be biologically active. Where a protein or polypeptide is biologically active, a portion of that protein or polypeptide that shares at least one biological activity of the protein or polypeptide can be referred to as a "biologically active" portion.

*Cation-independent mannose-6-phosphate receptor (CI-M6PR):* As used herein, the term "cation-independent mannose-6-phosphate receptor (CI-M6PR)" refers to a cellular receptor that binds mannose-6-phosphate (M6P) tags on acid hydrolase (e.g., alpha-N-acetyl-glucosaminidase) precursors in the Golgi apparatus that are destined for transport to the lysosome. In addition to mannose-6-phosphates, the CI-M6PR also binds certain proteins, including IGF-II. The CI-M6PR is also known as "CI-MPR", "M6P/IGF-II receptor", "CI-MPR/IGF-II receptor", "CD222", "MPR300", "IGF-II receptor" or "IGF2 Receptor." These terms and abbreviations thereof are used interchangeably herein.

*Enzyme replacement therapy (ERT)*: As used herein, the term "enzyme replacement therapy (ERT)" refers to any therapeutic strategy that corrects an enzyme deficiency by providing the missing enzyme. The missing enzyme can be provided by intrathecal administration. The missing enzyme can be provided by infusing into bloodsteam. Once administered, enzyme is taken up by cells and transported to the lysosome, where the enzyme acts to eliminate material that has accumulated in the lysosomes due to the enzyme deficiency. Typically, for lysosomal enzyme replacement therapy to be effective, the therapeutic enzyme is delivered to lysosomes in the appropriate cells in target tissues where the storage defect is manifest.

*Expression*: As used herein, "expression" of a nucleic acid sequence refers to one or more of the following events: (1) production of an RNA template from a DNA sequence (*e*.*g*., by transcription); (2) processing of an RNA transcript (*e*.*g*., by splicing, editing, 5' cap formation, and/or 3' end formation); (3) translation of an RNA into a polypeptide or protein; and/or (4) post-translational modification of a polypeptide or protein. In this application, the terms "expression" and "production," and grammatical equivalents, are used inter-changeably.

*Glycan*: As is known in the art and used herein "glycans" are sugars. Glycans can be monomers or polymers of sugar residues, but typically contain at least three sugars, and can be linear or branched. A glycan may include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2'-deoxyribose, phosphomannose, 6'sulfo N-acetylglucosamine, etc). The term "glycan" includes homo and heteropolymers of sugar residues. The term "glycan" also encompasses a glycan component of a glycoconjugate (*e*.*g*., of a glycoprotein, glycolipid, proteoglycan, etc.). The term also encompasses free glycans, including glycans that have been cleaved or otherwise released from a glycoconjugate.

*Glycoconjugate*: The term "glycoconjugate", as used herein, encompasses all molecules in which at least one sugar moiety is covalently linked to at least one other moiety. The term specifically encompasses all biomolecules with covalently attached sugar moieties, including for example N-linked glycoproteins, O-linked glycoproteins, glycolipids, proteoglycans, etc.

*Glycoprotein*: As used herein, the term "glycoprotein" refers to a protein that contains a peptide backbone covalently linked to one or more sugar moieties (i.e., glycans). As is understood by those skilled in the art, the peptide backbone typically comprises a linear chain of amino acid residues. The peptide backbone can span the cell membrane, such that it comprises a transmembrane portion, as well as an extracellular portion and/or an intracellular portion. A peptide backbone of a glycoprotein that spans the cell membrane can comprise an intracellular portion, a transmembrane portion, and an extracellular portion. The sugar moiety(ies) may be in the form of monosaccharides, disaccharides, oligosaccharides, and/or polysaccharides. The sugar moiety(ies) may comprise a single unbranched chain of sugar residues or may comprise one or more branched chains. Sugar moieties may include sulfate and/or phosphate groups. Alternatively or additionally, sugar moieties may include acetyl, glycolyl, propyl or other alkyl modifications. Alternatively or additionally, sugar moieties may be modified by diacetylation. Glycoproteins can contain O-linked sugar moieties; glycoproteins can contain N-linked sugar moieties. The sugar moiety can be specifically a N-linked glycan containing a mannose-6-phosphate residue. In certain embodiments, methods disclosed herein comprise a step of analyzing therapeutic glycoproteins, liberated fragments (e.g., glycopeptides) of cell surface glycoproteins, cell surface glycans attached to cell surface glycoproteins, peptide backbones of cell surface glycoproteins, fragments of such glycoproteins, glycans and/or peptide backbones, and combinations thereof for their glycosylation pattern and/or level of M6P phoshorylation.

*High mannose containing recombinant Naglu protein*: As used herein, the term "high mannose containing recombinant Naglu protein" refers to a recombinant Naglu (alpha-N-acetyl-glucosaminidase) protein containing one or more Man9(GlcNAc)2, Man8(GlcNAc)2, Man7(GlcNAc)2, Man6(GlcNAc)2, and/or Man5(GlcNAc)2 N-linked oligosaccharides, or combinations thereof.

*Homology*: As used herein, the term "homology" (or the corresponding adjective "homogous") refers to the overall relatedness of polymeric molecules, e.g., of nucleic acid molecules (e.g., DNA molecules and/or RNA molecules) and/or between polypeptide molecules. Polymeric molecules can be considered to be "homologous" to one another if their sequences are at least 25%, 30%, 35%, 40%, 45%, 50%, 55%, 60%, 65%, 70%, 75%, 80%, 85%, 90%, 95%, or 99% identical. Polymeric molecules can be considered to be "homologous" to one another if their sequences are at least 25%, 30%, 35%, 40%, 45%, 50%, 55%, 60%, 65%, 70%, 75%, 80%, 85%, 90%, 95%, or 99% similar.

*Identity*: As used herein, the term "identity" refers to the overall relatedness of polymeric molecules, e.g., of nucleic acid molecules (e.g., DNA molecules and/or RNA molecules) and/or of polypeptide molecules. Calculation of the percent identity of two nucleic acid sequences, for example, can be performed by aligning the two sequences for optimal comparison purposes (e.g., gaps can be introduced in one or both of a first and a second nucleic acid sequences for optimal alignment and non-identical sequences can be disregarded for comparison purposes). The length of a sequence aligned for comparison purposes can be at least 30%, at least 40%, at least 50%, at least 60%, at least 70%, at least 80%, at least 90%, at least 95%, or substantially 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4: 11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

*In Vitro:* As used herein, the term "*in vitro*" refers to events that occur in an artificial environment, *e.g.,* in a test tube or reaction vessel, in cell culture, *etc.,* rather than within a multi-cellular organism.

*In Vivo*: As used herein, the term "*in vivo*" refers to events that occur within a multi-cellular organism, such as a human and a non-human animal. In the context of cell-based systems, the term may be used to refer to events that occur within a living cell (as opposed to, for example, *in vitro* systems).

*Intrathecal administration*: As used herein, the term "intrathecal administration" or "intrathecal injection" refers to an injection into the spinal canal (intrathecal space surrounding the spinal cord). Various techniques may be used including, without limitation, lateral cerebroventricular injection through a burrhole or cisternal or lumbar puncture or the like. "Intrathecal administration" or "intrathecal delivery" can refer to IT administration or delivery via the lumbar area or region, i.e., lumbar IT administration or delivery. As used herein, the term "lumbar region" or "lumbar area" refers to the area between the third and fourth lumbar (lower back) vertebrae and, more inclusively, the L2-S1 region of the spine.

*Lysosomal enzyme*: As used herein, the term "lysosomal enzyme" refers to any enzyme that is capable of reducing accumulated materials in mammalian lysosomes or that can rescue or ameliorate one or more lysosomal storage disease symptoms. Lysosomal enzymes include both wild-type or modified lysosomal enzymes and can be produced using recombinant and synthetic methods or purified from nature sources.

*Lysosomal enzyme deficiency*: As used herein, "lysosomal enzyme deficiency" refers to a group of genetic disorders that result from deficiency in at least one of the enzymes that are required to break macromolecules (e.g., enzyme substrates) down to peptides, amino acids, monosaccharides, nucleic acids and fatty acids in lysosomes. As a result, individuals suffering from lysosomal enzyme deficiencies have accumulated materials in various tissues (e.g., CNS, liver, spleen, gut, blood vessel walls and other organs).

*Lysosomal Storage Disease*: As used herein, the term "lysosomal storage disease" refers to any disease resulting from the deficiency of one or more lysosomal enzymes necessary for metabolizing natural macromolecules. These diseases typically result in the accumulation of un-degraded molecules in the lysosomes, resulting in increased numbers of storage granules (also termed storage vesicles).

*Moiety*: As used herein, the term "moiety" refers to a part or functional group of a molecule (e.g., a glycan or a protein). The "moiety" is used interchangeably with the term "residue" herein.

*Mannose-6-phosphate-containing Naglu*: As used herein, the term "mannose-6-containing Naglu" refers to Naglu protein containing mannose-6-phosphate and includes situations where the mannose-6-phosphate (M6P) group is or is not the terminal moiety of a glycan bound to Naglu protein.

*Man9(GlcNAc)2, Man8(GlcNAc)2, Man7(GlcNAc)2, Man6(GlcNAc)2, and*/*or Man5(GlcNAc)2 N-linked oligosaccharides:* As used herein, these terms refer to glycans containing groups of 9, 8, 7, 6, and/or 5, respectively, mannose residues bound to a unit of two N-Acetylglucosamine residues, which in turn is linked to a protein via a nitrogen atom.

*N-acetylglucosamine-1-phosphotransferase (GNPT) protein*: As used herein, the term N-acetylglucosamine-1-phosphotransferase (GNPT) protein refers to enzymes capable of catalyzing the transfer of N-acetylglucosamine-1-phosphate from UDP-GlcNAc to mannose residues on lysosomal hydrolases (e.g., alpha-N-acetyl-glucosaminidase).

*Nucleic acid*: As used herein, the term "nucleic acid," in its broadest sense, refers to any compound and/or substance that is or can be incorporated into a polynucleotide chain. A nucleic acid can be a compound and/or substance that is or can be incorporated into a polynucleotide chain via a phosphodiester linkage. "Nucleic acid" can refer to individual nucleic acid residues (e.g., nucleotides and/or nucleosides). "Nucleic acid" can refer to a polynucleotide chain comprising individual nucleic acid residues. "Nucleic acid" can encompass RNA as well as single and/or double-stranded DNA and/or cDNA. Furthermore, the terms "nucleic acid," "DNA," "RNA," and/or similar terms include nucleic acid analogs, *i.e.,* analogs having other than a phosphodiester backbone. For example, the so-called "peptide nucleic acids," are known in the art and have peptide bonds instead of phosphodiester bonds in the backbone. The term "nucleotide sequence encoding an amino acid sequence" includes all nucleotide sequences that are degenerate versions of each other and/or encode the same amino acid sequence. Nucleotide sequences that encode proteins and/or RNA may include introns. Nucleic acids can be purified from natural sources, produced using recombinant expression systems and optionally purified, chemically synthesized, *etc.* Where appropriate, *e.g.,* in the case of chemically synthesized molecules, nucleic acids can comprise nucleoside analogs such as analogs having chemically modified bases or sugars, backbone modifications, *etc.* A nucleic acid sequence is presented in the 5' to 3' direction unless otherwise indicated. A nucleic acid can be or can comprise natural nucleosides (*e*.*g*., adenosine, thymidine, guanosine, cytidine, uridine, deoxyadenosine, deoxythymidine, deoxyguanosine, and deoxycytidine); nucleoside analogs (*e.g., 2-*aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g.,* methylated bases); intercalated bases; modified sugars (*e.g.,* 2'-fluororibose, ribose, 2'-deoxyribose, arabinose, and hexose); and/or modified phosphate groups (*e*.*g*., phosphorothioates and 5'-*N*-phosphoramidite linkages). In some instances, the present disclosure is specifically directed to "unmodified nucleic acids," meaning nucleic acids (*e.g.,* polynucleotides and residues, including nucleotides and/or nucleosides) that have not been chemically modified in order to facilitate or achieve delivery.

*Pharmaceutically acceptable*: The term "pharmaceutically acceptable" as used herein, refers to substances that, within the scope of sound medical judgment, are suitable for use in contact with the tissues of human beings and animals without excessive toxicity, irritation, allergic response, or other problem or complication, commensurate with a reasonable benefit/risk ratio.

*Polypeptide*: As used herein, a "polypeptide", generally speaking, is a string of at least two amino acids attached to one another by a peptide bond. A polypeptide may include at least 3-5 amino acids, each of which is attached to others by way of at least one peptide bond. Those of ordinary skill in the art will appreciate that polypeptides sometimes include "non-natural" amino acids or other entities that nonetheless are capable of integrating into a polypeptide chain, optionally.

*Protein:* As used herein, the term "protein" of "therapeutic protein" refers to a polypeptide (*i.e.,* a string of at least two amino acids linked to one another by peptide bonds). Proteins may include moieties other than amino acids (*e*.*g*., may be glycoproteins, proteoglycans, *etc.*) and/or may be otherwise processed or modified. Those of ordinary skill in the art will appreciate that a "protein" can be a complete polypeptide chain as produced by a cell (with or without a signal sequence), or can be a characteristic portion thereof. Those of ordinary skill will appreciate that a protein can sometimes include more than one polypeptide chain, for example linked by one or more disulfide bonds or associated by other means. Polypeptides may contain 1-amino acids, d-amino acids, or both and may contain any of a variety of amino acid modifications or analogs known in the art. Useful modifications include, *e*.*g*., terminal acetylation, amidation, methylation, *etc.* In some embodiments, proteins may comprise natural amino acids, non-natural amino acids, synthetic amino acids, and combinations thereof. The term "peptide" is generally used to refer to a polypeptide having a length of less than about 100 amino acids, less than about 50 amino acids, less than 20 amino acids, or less than 10 amino acids. Proteins can be antibodies, antibody fragments, biologically active portions thereof, and/or characteristic portions thereof.

*Recombinant*: As used herein, the term "recombinant" refers to DNA molecules, peptides, proteins or cells that are generated using recombinant DNA technology, as the term is commonly understood by the person gaving ordinary skill in the art.

*Subject*: As used herein, the term "subject" refers to a human or any non-human animal (e.g., mouse, rat, rabbit, dog, cat, cattle, swine, sheep, horse or primate). A human includes pre- and post-natal forms. A subject may be a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially*: As used herein, the term "substantially" refers to a an amount greater than 80%, 85%, 90%, 95%, 96%, 97%, 98%, 99% of the total amount.

*Substantially high mannose containing recombinant Nagluprotein*: As used herein, the term "substantially high mannose containing recombinant Naglu protein" refers to a recombinant Naglu protein containing glycans greater than 80%, of which is Man9(GlcNAc)2, Man8(GlcNAc)2, Man7(GlcNAc)2, Man6(GlcNAc)2, and/or Man5(GlcNAc)2 N-linked oligosaccharides, or combinations thereof.

*Treatment*: As used herein, the term "treatment" (also "treat" or "treating") refers to any administration of a therapeutic protein (e.g., lysosomal enzyme) that partially or completely alleviates, ameliorates, relieves, inhibits, delays onset of, reduces severity of and/or reduces incidence of one or more symptoms or features of a particular disease, disorder, and/or condition (e.g., Hunters syndrome, Sanfilippo B syndrome). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition.

*Therapeutically effective amount*: As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.

### DETAILED DESCRIPTION

The present disclosure provides mannose-6-phosphate (M6P)-containing recombinant alpha-N-acetyl-glucosaminidase (Naglu) as defined in the claims, which is suitable for effective enzyme replacement therapy of Sanfilippo syndrome type B and methods of making and using the same. The present invention provides a method of producing M6P-containing recombinant Naglu by providing a high mannose containing Naglu protein which has been generated from mammalian or non-mammalian cells using recombinant DNA technology; and contacting the high mannose containing Naglu protein with N-acetylglucosamine-1-phosphotransierase (GNPT) under conditions that permit phosphorylation of one or more mannose residues on the recombinant Naglu protein, wherein conditions that permit phosphorylation comprise a greater than 1:1 GNPT to Naglu molar ratio such that GNPT is in excess of Naglu. The high mannose containing Naglu protein may be produced from cells (e.g., human or CHO cells) cultured in the presence of an alpha-mannosidase I inhibitor.

Various aspects of the invention are described in further detail in the following subsections. The use of subsections is not meant to limit the invention. Each subsection may apply to any aspect of the invention. In this application, the use of "or" means "and/or" unless stated otherwise.

### Naglu Protein

Alpha-N-acetyl-glucosaminidase (Naglu) aids in the stepwise degradation of the heparan sulfate in lysosomes by removing GlcNAc (N-acetyl-glucosamine) from the non-reducing end of this glycosaminoglycan. A lack of Naglu leads to incomplete breakdown of heparan sulfate and the subsequent accumulation in lysosomes of the incomplete breakdown products. The absence of Naglu causes the fatal neurodegenerative disease Sanfilippo Type III B.

A suitable Naglu protein according to the present invention can be any molecule that can substitute for naturally-occurring Naglu protein activity or rescue one or more phenotypes or symptoms associated with Naglu-deficiency. In some embodiments, a Naglu protein suitable for the invention is a polypeptide having an N-terminus and a C-terminus and an amino acid sequence substantially similar or identical to the mature human Naglu protein.

Typically, human Naglu is produced as a precursor molecule that is processed to a mature form. This process generally occurs by removing the 23 amino acid signal peptide as the protein enters the endoplasmic reticulum. Typically, the precursor form is also referred to as full-length precursor or full-length Naglu protein, which contains 743 amino acids. The N-terminal 23 amino acids are cleaved as the precursor protein enters the endoplasmic reticulum, resulting in a mature form. Thus, it is contemplated that the N-terminal 23 amino acids are generally not required for Naglu protein activity. The amino acid sequences of the mature form (**SEQ ID NO:1**) and full-length precursor (**SEQ ID NO:2**) of a typical wild-type or naturally-occurring human Naglu protein are shown in Table 1 below.

**Table 1. Mature and Full-length Precursor Naglu Protein**

| | |
|---|---|
| **Mature Form** | DEAREAAAVRALVARLLGPGPAADFSVSVERALAAKPGLDTYSLGGGGAARVRV RGSTGVAAAAGLHRYLRDFCGCHVAWSGSQLRLPRPLPAVPGELTEATPNRYRY |
| | YQNVCTQSYSFVWWDWARWEREIDWMALNGINLALAWSGQEAIWQRVYLALGLT QAEINEFFTGPAFLAWGRMGNLHTWDGPLPPSWHIKQLYLQHRVLDQMRSFGMT PVLPAFAGHVPEAVTRVFPQVNVTKMGSWGHFNCSYSCSFLLAPEDPIFPIIGS LFLRELIKEFGTDHIYGADTFNEMQPPSSEPSYLAAATTAVYEAMTAVDTEAVW LLQGWLFQHQPQFWGPAQIRAVLGAVPRGRLLVLDLFAESQPVYTRTASFQGQP FIWCMLHNFGGNHGLFGALEAVNGGPEAARLFPNSTMVGTGMAPEGISQNEVVY SLMAELGWRKDPVPDLAAWVTSFAARRYGVSHPDAGAAWRLLLRSVYNCSGEAC RGHNRSPLVRRPSLQMNTSIWYNRSDVFEAWRLLLTSAPSLATSPAFRYDLLDL TRQAVQELVSLYYEEARSAYLSKELASLLRAGGVLAYELLPALDEVLASDSRFL LGSWLEQARAAAVSEAEADFYEQNSRYQLTLWGPEGNILDYANKQLAGLVANYY TPRWRLFLEALVDSVAQGIPFQQHQFDKNVFQLEQAFVLSKQRYPSQPRGDTVD LAKKIFLKYYPRWVAGSW (SEQ ID NO.:1) |
| **Full-Length Precursor** |  (SEQ ID NO.:2) |

Thus, in some embodiments, Naglu protein suitable for the present invention is mature human Naglu protein (**SEQ ID NO:1**). In some embodiments, a suitable Naglu protein may be a homologue or an orthologue of the mature human Naglu protein from a different species (e.g., mouse, rat, sheep, pig, dog, etc.). In other embodiments, a suitable Naglu protein may be a functional variant of the mature human Naglu protein. A functional variant of the mature human Naglu protein may be a modified mature human Naglu protein containing one or more amino acid substitutions, deletions, and/or insertions as compared to a wild-type or naturally-occurring Naglu protein (e.g., **SEQ ID NO:1**), while substantially retaining the biological ativity of Naglu protein. Thus, in some embodiments, a Naglu protein suitable for the present invention is substantially homologous to mature human Naglu protein (**SEQ ID NO:1**). In some embodiments, a Naglu protein suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to **SEQ ID NO:1**. In some embodiments, a Naglu protein suitable for the present invention is substantially identical to mature human Naglu protein (**SEQ ID NO:1**). In some embodiments, a Naglu protein suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to **SEQ ID NO:1**. In some embodiments, a Naglu protein suitable for the present invention contains a fragment or a portion of mature human Naglu protein.

Alternatively, a Naglu protein suitable for the present invention is full-length Naglu protein. In some embodiments, a Naglu protein suitable may be a homologue or an orthologue of full-length human Naglu protein from a different species (e.g. mouse, rat, sheep, pig, dog, etc.). In some embodiments, a suitable Naglu protein may be a functional variant of the full-length human Naglu protein containing one or more amino acid substitutions, deletions, and/or insertions as compared to a wild-type or naturally-occurring full-length Naglu protein (e.g., **SEQ ID NO:2**), while retaining substantial Naglu protein activity. Thus, In some embodiments, Naglu protein suitable for the present invention is substantially homologous to full-length human Naglu protein (**SEQ ID NO:2**). In some embodiments, a Naglu protein suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to **SEQ ID NO:2**. In some embodiments, a Naglu protein suitable for the present invention is substantially identical to **SEQ ID NO:2**. In some embodiments, a Naglu protein suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to **SEQ ID NO:2**. In some embodiments, a Naglu protein suitable for the present invention contains a fragment or a portion of full-length human Naglu protein. As used herein, a full-length Naglu protein typically contains signal peptide sequence.

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology. Two sequences can be considered to be substantially homologous if at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or more of their corresponding residues are homologous over a relevant stretch of residues. The relevant stretch can be a complete sequence. The relevant stretch can be at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 225, at least 250, at least 275, at least 300, at least 325, at least 350, at least 375, at least 400, at least 425, at least 450, at least 475, at least 500 or more residues.

The phrase "substantial identity" is used herein to refer to a comparison between amino acid or nucleic acid sequences. As will be appreciated by those of ordinary skill in the art, two sequences are generally considered to be "substantially identical" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul et al., Nucleic Acids Res. 25:3389-3402, 1997; Baxevanis et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying identical sequences, the programs mentioned above typically provide an indication of the degree of identity. Two sequences can be considered to be substantially identical if at least 50%, 55%, 60%, 65%, 70%, 75%, 80%, 85%, 90%, 91%, 92%, 93%, 94%, 95%, 96%, 97%, 98%, 99% or more of their corresponding residues are identical over a relevant stretch of residues. The relevant stretch can be a complete sequence. The relevant stretch can be at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more residues.

### Obtaining High Mannose Containing Recombinant Naglu Protein

Like many other lysosomal enzymes, a Naglu protein is normally targeted to lysosomes via the CI-M6P receptor mediated pathway. Therefore, a recombinant Naglu protein normally requires addition of mannose-6-phosphate (M6P) groups by GNPT to the N-linked, high mannose type carbohydrates present on the protein. As shown in the Examples section, the present inventors discovered unexpectedly that recombinant Naglu protein produced in conventional cell culture system completely lacks high mannose glycans, which explains why it was virtually impossible to produce M6P-containing recombinant Naglu protein prior to the present invention. The present inventors further demonstrated that high mannose containing recombinant Naglu protein may be successfully produced from cells cultured in the presence of an alpha-mannosidase I inhibitor.

### Alpha-Mannosidase I Inhibitors

Alpha-mannosidase I inhibitors prevent mannosidases from trimming mannose residues from the precursor glycoprotein, which causes cells to secrete high mannose-containing glycoproteins. In some embodiments, the high mannose containing recombinant Naglu protein is produced from cells cultured in the presence of an alpha-mannosidase I inhibitor. In some embodiments, the alpha-mannosidase I inhibitor is selected from the group consisting of deoxymannojirimycin, kifunensine, D-Mannonolactam amidrazone, N-butyl-dexoymannojirimycin, and combinations thereof.

In particular embodiments, a suitable alpha-mannosidase I inhibitor is kifunensine. Kifunensine is an inhibitor of mannosidase I and weakly inhibits arylmanosidase. It shows no inhibitory action against mannosidase II.

### Host Cells

Suitable host cells can be derived from a variety of organisms, including, but not limited to, mammals, plants, birds (e.g., avian systems), insects, yeast, and bacteria. In some embodiments, host cells are mammalian cells. Any mammalian cell susceptible to cell culture, and to expression of polypeptides, may be utilized in accordance with the present invention as a host cell. Non-limiting examples of mammalian cells that may be used in accordance with the present invention include human embryonic kidney 293 cells (HEK293), HeLa cells; BALB/c mouse myeloma line (NSO/1, ECACC No: 85110503); human retinoblasts (PER.C6 (CruCell, Leiden, The Netherlands)); monkey kidney CV1 line transformed by SV40 (COS-7, ATCC CRL 1651); human fibrosarcomacell line (e.g., HT-1080); human embryonic kidney line (293 or 293 cells subcloned for growth in suspension culture, Graham et al., J. Gen Virol., 36:59 (1977)); baby hamster kidney cells (BHK, ATCC CCL 10); Chinese hamster ovary cells +/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 (1982)); MRC 5 cells; FS4 cells; a human hepatoma line (Hep G2), human cell line CAP and AGE1.HN, and Glycotope's panel.

In some embodiments, host cells are non-mammalian cells. Suitable non-mammalian cells include prokaryotes, archae, yeast and other eukaryotic cells. Non-limiting examples of non-mammalian host cells suitable for the present invention include yeast cells and cell lines derived from *Pichia pastoris, Pichia methanolica*, *Pichia angusta*, *Schizosacccharomyces pombe, Saccharomyces cerevisiae,* and *Yarrowia lipolytica;* insect cells and cell lines derived from *Sodoptera frugiperda, Trichoplusis ni*, *Drosophila melanogaster* and *Manduca sexta*; and cells nd cell lines derived from *Escherichia coli, Salmonella typhimurium, Bacillus subtilis, Bacillus lichenifonnis*, *Bacteroides fragilis, Clostridia perfringens*, *Clostridia difficile;* and amphibian cells and ecll lines derived *Xenopus Laevis.*

Vectors for expression of Naglu and/or other components of the disclosure include plasmid vectors, double or single-stranded RNA or DNA viral vectors. Vectors may be introduced directly into cells as DNA or through indirect use of phages and viruses.

### Culture Conditions

In some embodiments, the presently claimed method further comprises producing recombinant high mannose containing Naglu protein using a standard cell culture system in the presence of alpha mannosidase 1 inhibitors. For example, a suitable alpha mannosidase 1 inhibitor (e.g., kifunensine) may be added to the cell culture medium at various concentrations, for example, at a concentration from 0.001 to 0.01, from 0.01 to 0.05, from 0.05 to 0.1, from 0.1 to 0.5, from 0.5 to 1, from 1 to 2, from 2 to 5, from 5 to 10, from 10 to 20, from 20 to 100, or from 100 to 1000 mg/l. A suitable alpha mannosidase 1 inhibitor (e.g., kifunensine) may also be added to the cell culture medium at a concentration of 0.001, 0.1, 0.5, 1, 2, 3, 4, 5, 6, 7, 8, 9, 10, 50, or 100 mg/l.

In some embodiments, the cell culture system is used for microscale production of a recombinant high mannose containing Naglu protein. In some embodiments, the cellular culture system is used for large scale production. Typical large-scale procedures for producing a recombinant polypeptide of interest include batch cultures and fed-batch cultures. Batch culture processes traditionally comprise inoculating a large-scale production culture with a seed culture of a particular cell density, growing the cells under conditions (e.g., suitable culture medium, pH, and temperature) conducive to cell growth, viability, and/or productivity, harvesting the culture when the cells reach a specified cell density, and purifying the expressed polypeptide. Fed-batch culture procedures include an additional step or steps of supplementing the batch culture with nutrients and other components that are consumed during the growth of the cells. In some embodiments, a large-scale production method according to the present invention uses a fed-batch culture system.

In some embodiments, bioreactors are used to produce recombinant high mannose containing Naglu protein. Bioreactors may be perfusion, batch, fed-batch, repeated batch, or continuous (e.g., a continuous stirred-tank reactor models), for example. Typically, the bioreactors comprise at least one vessel designed and configured to house medium (e.g., a chemically defined nutrient medium). The vessel also typically comprises at least one inlet designed and configured to flow fresh nutrient medium and/or to add alpha mannosidase 1 inhibitors into the vessel. The vessel also typically comprises at least one outlet designed and configured to flow waste medium out of the vessel. In some embodiments, the vessel may further comprise at least one filter designed and configured to minimize the extent to which isolated cells in the vessel are passed out through the at least one outlet with waste medium. The bioreactor may also be fitted with one or more other components designed to maintain conditions suitable for cell growth. For example, the bioreactor may be fitted with one or more circulation or mixing devices designed and configured to circulate or mix the nutrient medium within the vessel. Vessels of any appropriate size may be used in the bioreactors. In some embodiments, the vessel is designed and configured to contain up to 1 L, up to 10 L, up to 100 L, up to 500 L, up to 1000 l, up to 1500 l, up to 2000 l, or more of the nutrient medium. In some embodiments, the volume of the production bioreactor is at least 10 l, at least 50 l, 100 l, at least 200 l, at least 250 l, at least 500 l, at least 1000 l, at least 1500 l, at least 2000 l, at least 2500 l, at least 5000 l, at least 8000 l, at least 10,000 l, or at least 12,000 l, or more, or any volume in between.

In certain embodiments of the present invention, the practitioner may find it beneficial to periodically monitor particular conditions of the growing cell culture. Monitoring cell culture conditions allows the practitioner to determine whether the cell culture is producing recombinant polypeptide or protein at suboptimal levels or whether the culture is about to enter into a suboptimal production phase.

Typically, it is desirable to monitor the level and/or profile/pattern of the glycosylation of a recombinant Naglu protein. In particular, analysis can be performed to monitor high mannose structures present on the protein.

### Purification of High Mannose Containing Recombinant Naglu Protein

Various methods may be used to purify or isolate high mannose containing recombinant Naglu protein produced according to various methods described herein. For example, high mannose containing recombinant Naglu protein may be isolated and purified by standard methods including, but not limited to, chromatography (e.g., ion exchange, affinity, size exclusion, and hydroxyapatite chromatography), gel filtration, centrifugation, or differential solubility, ethanol precipitation or by any other available technique for the purification of proteins (see, e.g., Scopes, Protein Purification Principles and Practice 2nd Edition, Springer-Verlag, New York, 1987; Higgins, S. J. and Hames, B. D. (eds.), Protein Expression: A Practical Approach, Oxford Univ Press, 1999; and Deutscher, M. P., Simon, M. I., Abelson, J. N. (eds.), Guide to Protein Purification: Methods in Enzymology (Methods in Enzymology Series, Vol 182), Academic Press, 1997). For immunoaffinity chromatography in particular, the protein may be isolated by binding it to an affinity column comprising antibodies that were raised against that protein and were affixed to a stationary support. Protease inhibitors such as phenyl methyl sulfonyl fluoride (PMSF), leupeptin, pepstatin or aprotinin may be added at any or all stages in order to reduce or eliminate degradation of the polypeptide or protein during the purification process.

### Provided Recombinant High Mannose Containing Naglu Protein

In the method of the present invention, high mannose containing recombinant Naglu is provided. As used herein, the term "high mannose containing recombinant Naglu protein" refers also to any recombinant Naglu protein that contains substantially Man9(GlcNAc)2, Man8(GlcNAc)2, Man7(GlcNAc)2, Man6(GlcNAc)2, and/or Man5(GlcNAc)2 N-linked oligosaccharides. For example, the combined Man9(GlcNAc)2, Man8(GlcNAc)2, Man7(GlcNAc)2, Man6(GlcNAc)2, and/or Man5(GlcNAc)2 N-linked oligosaccharides on a high mannose containing recombinant Naglu protein may constitute greater than 70%, 75%, 80%, 85%, 90%, 95%, 96%, 97%, 98%, or 99% of the total oligosaccharides present on the protein.

In some embodiments, a high mannose containing recombinant Naglu protein contains substantially Man9(GlcNAc)2, Man8(GlcNAc)2, Man7(GlcNAc)2, and/or Man6(GlcNAc)2 N-linked oligosaccharides. For example, the combinaed Man9(GlcNAc)2, Man8(GlcNAc)2, Man7(GlcNAc)2, and/or Man6(GlcNAc)2 N-linked oligosaccharides on a high mannose containing recombinant Naglu protein may constitute greater than 70%, 75%, 80%, 85%, 90%, 95%, 96%, 97%, 98%, or 99% of the total oligosaccharides present on the protein.

In some embodiments, a high mannose containing recombinant Naglu protein contains substantially Man9(GlcNAc)2, Man8(GlcNAc)2, and/or Man7(GlcNAc)2 N-linked oligosaccharides. For example, the combinaed Man9(GlcNAc)2, Man8(GlcNAc)2, and/or Man7(GlcNAc)2 N-linked oligosaccharides on a high mannose containing recombinant Naglu protein may constitute greater than 70%, 75%, 80%, 85%, 90%, 95%, 96%, 97%, 98%, or 99% of the total oligosaccharides present on the protein.

In some embodiments, a high mannose containing recombinant Naglu protein contains substantially Man9(GlcNAc)2 and/or Man8(GlcNAc)2 N-linked oligosaccharides. For example, the combinaed Man9(GlcNAc)2 and/or Man8(GlcNAc)2 N-linked oligosaccharides on a high mannose containing recombinant Naglu protein may constitute greater than 70%, 75%, 80%, 85%, 90%, 95%, 96%, 97%, 98%, or 99% of the total oligosaccharides present on the protein.

In some embodiments, a high mannose containing recombinant Naglu protein contains substantially Man9(GlcNAc)2 N-linked oligosaccharides. In particular embodiments, Man9(GlcNAc)2 N-linked oligosaccharides on a high mannose containing recombinant Naglu protein may constitute greater than 70%, 75%, 80%, 85%, 90%, 95%, 96%, 97%, 98%, or 99% of the total oligosaccharides present on the protein.

In some embodiments, a high mannose containing recombinant Naglu protein may contain an increase in the number and/or level of high mannose structures, such as Man9(GlcNAc)2, Man8(GlcNAc)2, Man7(GlcNAc)2, Man6(GlcNAc)2, and/or Man5(GlcNAc)2 N-linked oligosaccharides, compared to recombinant Naglu protein not produced according to the present invention. In some embodiments, the increase in Man9(GlcNAc)2 oligosaccharides is 1, 2, 3, 4, 5, 6, 7, 8, 9, 10, 11, 12, 13, 14,15, 20, 25, 30, 35, 40, 45, 50, 55, 60, 65, 70, 75, 80, 85, or 90 percent. In some embodiments, the increase in Man8(GlcNAc)2 oligosaccharides is 1, 2, 3, 4, 5, 6, 7, 8, 9, 10, 11, 12, 13, 14,15, 20, 25, 30, 35, 40, 45, 50, 55, 60, 65, 70, 75, 80, 85, or 90 percent. In some embodiments, the increase in Man7(GlcNAc)2 oligosaccharides is 1, 2, 3, 4, 5, 6, 7, 8, 9, 10, 11, 12, 13, 14,15, 20, 25, 30, 35, 40, 45, 50, 55, 60, 65, 70, 75, 80, 85, or 90 percent. In some embodiments, the increase in Man6(GlcNAc)2 oligosaccharides is 1, 2, 3, 4, 5, 6, 7, 8, 9, 10, 11, 12, 13, 14,15, 20, 25, 30, 35, 40, 45, 50, 55, 60, 65, 70, 75, 80, 85, or 90 percent. In some embodiments, the increase in Man5(GlcNAc)2 oligosaccharides is 1, 2, 3, 4, 5, 6, 7, 8, 9, 10, 11, 12, 13, 14, 15, 20, 25, 30, 35, 40, 45, 50, 55, 60, 65, 70, 75, 80, 85, or 90 percent.

The extent as to which Naglu carrries these and other types of glycans can be determined by methodologies that are routinely used in the art. Non-limiting examples of such methods are disclosed in the Examples below.

### Generation of M6P-Containing Recombinant Naglu Protein

Typically, one or more M6P groups are added to high mannose containing N-linked oligosaccharides in a two-step process. First, N-acetylglucosamine 1-phosphate is attached to the C₆-hydroxyl group of a mannose residue, followed by removal of N-acetylglucosamine. The first reaction is catalyzed by the UDP-GlcNAc:lysosomal enzyme N-acetylglucosamine-1-phosphotransferase (GNPT), while the second is catalyzed by an alpha-N-acetyl-glucosaminidase. GNPT is a hexameric complex containing three distinct subunits (α2, β2, γ2). The lysosomal substrate protein binds within the active cleft of GNPT, where GNPT catalyzes the addition of N-acetylglucosamine 1-phosphate to the protein, making use of uridine diphosphate N-acetylglucosamine (UDP-GlcNAc) as the molecular substrate.

Thus, the present invention provides a method of producing mannose-6-phosphate (M6P)-containing Naglu including a step of contacting the high mannose containing recombinant Naglu protein with N-acetylglucosamine-1-phospho transferase (GNPT) under conditions that permit phosphorylation of one or more mannose residues on the recombinant Naglu protein, wherein conditions that permit phosphorylation comprise a greater than 1:1 GNPT to Naglu molar ratio such that GNPT is in excess of Naglu. In some embodiments, such a contacting and phosphorylation step is carried out in vitro. For example, purified recombinant high mannose-containing Naglu protein described herein may be mixed with purified GNPT in a reaction vessel. Purified GNPT may be produced recombinantly using standard cell culture system. GNPT may also be purified by extraction from organs, blood or other bodily tissues or from non-human transgenic animals expressing a human GNPT. Recombinant high mannose-containing Naglu protein described herein may also be contacted with GNPT in a tissue culture cell, for example by over-expressing both proteins in said cell.

### GNPT

In some embodiments, a GNPT enzyme suitable for the present invention is human GNPT protein (**SEQ ID NO:3 and/or 4**). In some embodiments, a suitable GNPT enzyme may be a homologue or an ortholog of human GNPT protein. In some embodiments, the GNPT protein may be a modified human GNPT protein containing one or more amino acid substitutions, deletions, and/or insertions as compared to a wild-type or naturally-occurring GNPT protein (e.g., **SEQ ID NO:3 and/or 4**), while retaining the substantial biological activity of the wild-type or naturally-occurring GNPT protein. Thus, in some embodiments, a GNPT enzyme suitable for the present invention is substantially homologous to human GNPT protein (**SEQ ID NO:3 and/or 4**)**.** In some embodiments, a GNPT enzyme suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to **SEQ ID NO:3 and/or 4.** In some embodiments, a GNPT enzyme suitable for the present invention is substantially identical to human GNPT protein (**SEQ ID NO:3 and/or 4**)**.** In some embodiments, a GNPT enzyme suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to **SEQ ID NO:4 and/or 4**. In some embodiments, a GNPT enzyme suitable for the present invention contains a fragment or a portion of mature human GNPT protein.

**Table 2. Human GNPT Protein**

| | |
|---|---|
| **Human N-acetylglucosamine-1-phosphotransferase (*Alpha and Beta subunits*)** | (SEQ ID NO.:3) |
| ***Underlined Region-TGRQLK -is alpha/beta cleavage site** | |
| **Human N-acetylglucosamine-1-phosphotransferase (*Gamma Subunit*)** |  (SEQ ID NO.:4) |

In some embodiments, a GNPT enzyme suitable for the present invention is a modified soluble human GNPT protein (sGNPT) (**SEQ ID NO:5**) (Kudo M. & Canfield W. M., J. Biol. Chem. 281:11761-11768 (2006)). In some embodiments, a GNPT enzyme suitable for the present invention is substantially homologous to the modified soluble human GNPT protein (alpha/beta subunits) (**SEQ ID NO:5**). In some embodiments, a GNPT enzyme suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to **SEQ ID NO:5.** In some embodiments, a GNPT enzyme suitable for the present invention is substantially identical to **SEQ ID NO:5.** In some embodiments, a GNPT enzyme suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to **SEQ ID NO:5**. In some embodiments, a GNPT enzyme suitable for the present invention contains a fragment or a portion of a sGNPT (alpha/beta subunits) (**SEQ ID NO:5**). In some embodiments, a GNPT enzyme suitable for the present invention is homologous and/or identical with respect to the gamma subunit (**SEQ ID NO:4**) of sGNPT to varying degrees, as described in relation to the alpha/beta subunits above.

**Table 3. Modified Soluble Human GNPT Protein**

| | |
|---|---|
| **Soluble human N-acetylglucosamine-1-phosphotransferase (sGNPT) (*Alpha and Beta subunits*)** | METDTLLLWVLLLWVPGSTGDEDQVDPRLIDGKWSRDQYHVLFDSYRDNIAGKS FQNRLCLPMPIDVVYTWVNGTDLELLKELQQVREQMEEEQKAMREILGKNTTEP TKKSEKQLECLLTHCIKVPMLVLDPALPANITLKDLPSLYPSFHSASDIFNVAK PKNPSTNVSVVVFDSTKDVEDAHSGLLKGNSRQTVWRGYLTTDKEVPGLVLMQD LAFLSGFPPTFKETNQLKTKLPENLSSKVKLLQLYSEASVALLKLNNPKDFQEL NKQTKKNMTIDGKELTISPAYLLWDLSAISQSKQDEDISASRFEDNEELRYSLR SIERHAPWVRNIFIVTNGQIPSWLNLDNPRVTIVTHQDVFRNLSHLPTFSSPAI ESHIHRIEGLSQKFIYLNDDVMFGKDVWPDDFYSHSKGQKVYLTWPVPNCAEGC PGSWIKDGYCDKACNNSACDWDGGDCSGNSGGSRYIAGGGGTGSIGVGQPWQFG GGINSVSYCNQGCANSWLADKFCDQACNVLSCGFDAGDCGQDHFHELYKVILLP NQTHYIIPKGECLPYFSFAEVAKRGVEGAYSDNPIIRHASIANKWKTIHLIMHS GMNATTIHFNLTFQNTNDEEFKMQITVEVDTREGPKLNSTAQKGYENLVSPITL LPEAEILFEDIPKEKRFPKFKRHDVNSTRRAQEEVKIPLVNISLLPKDAQLSLN TLDLQLEHGDITLKGYNLSKSALLRSFLMNSQHAKIKNQAIITDETNDSLVAPQ EKQVHKSILPNSLGVSERLQRLTFPAVSVKVNGHDQGQNPPLDLETTARFRVET HTQKTIGGNVTKEKPPSLIVPLESQMTKEKKITGKEKENSRMEENAENHIGVTE VLLGRKLQHYTDSYLGFLPWEKKKYFQDLLDEEESLKTQLAYFTDSKNRARYKR DTFADSLRYVNKILNSKFGFTSRKVPAHMPHMIDRIVMQELQDMFPEEFDKTSF HKVRHSEDMQFAFSYFYYLMSAVQPLNISQVFDEVDTDQSGVLSDREIRTLATR IHELPLSLQDLTGLEHMLINCSKMLPADITQLNNIPPTQESYYDPNLPPVTKSL VTNCKPVTDKIHKAYKDKNKYRFEIMGEEEIAFKMIRTNVSHVVGQLDDIRKNP RKFVCLNDNIDHNHKDAQTVKAVLRDFYESMFPIPSQFELPREYRNRFLHMHEL QEWRAYRDKL (SEQ ID NO.:5) |

The amimo acid sequence METDTLLLWVLLLWVPGSTGD in SEQ ID No.: 5 (underlined in Table 3 above) corresponds to the mouse immunoglobulin kappa chain signal peptide and cleavage site and this sequence replaces the putative signal anchor/transmembrane domain at the amino terminus (amino acids 1-44) of native human GNPT alpha/beta (i.e., MLFKLLQRQTYTCLSHRYGLYVCFLGVVVTIVSAFQFGEVVLE).

The amino acid sequence EDQVDPRLIDGK in SEQ ID No.: 5 (underlined in Table 3 above) between the signal peptide and mature alpha subunit is a HPC4 epitope that aids in the purification of sGNPT.

The alpha and beta cleavage sequence in SEQ ID NO.: 5 is RARYKR (underlined in Table 3 above) rather than TGRQLK as in the native human protein.

The transmembrane domain and ER retention signal from the c-terminal end of the beta subunit of the native human GNPT (KFWTHCVLATLIMFTIFSFFAEQLIALKRK IFPRRRIHKEASPNRIRV) is removed to make sGNPT more soluble and thus easier to purifiy.

### Phosphorylation of High Mannose Containing Naglu

Purified recombinant high mannose containing Naglu is then subjected to treatment with GNPT under in vitro conditions favorable for GNPT catalyzing the addition of M6P to Naglu. High mannose structures, typically six to nine mannose molceules with two N-acetuylglucosamine molecules, are recognized on Naglu and subsequently phosphorylated by GNPT. High mannose Naglu is treated with an excess GNPT, which catalyzes transfer of N-acetylglucosamine-1-phosphate from UDP-GlcNAc to the 6' position of α1,2 -linked (or other outer mannoses) on Naglu. In some embodiments, Naglu can then be treated with an alpha-N-acetyl-glucosaminidase to remove the N-acetylglucosamine residue, which produces the terminal M6P moiety.

High mannose containing Naglu protein and GNPT may be mixed at various ratios. For example, high mannose containing Naglu protein and GNPT may be mixed at an about equal molar ratio (i.e.,an about 1:1 GNPT to Naglu molar ratio). An excess amount of GNPT is used (i.e., a greater than 1:1 GNPT to Naglu molar ratio). For example, GNPT and Naglu protein may be mixed at a or a greater than about 2:1, 3:1, 4:1, 5:1, 10:1, 20:1, 30:1, 40:1, 50:1, 100:1, 200:1, 300:1, 400:1, 500:1, or 1,000:1 GNPT to Naglu molar ratio.

In some embodiments, phosphorylation of Naglu protein by GNPT may take place in a cell based system. For example, phosphorylation of Naglu protein may take place by over-expressing Naglu protein and GNPT protein in suitable cells simultaneously in the presence ofan alpha mannosidase 1 inhibitor (e.g., kifunensine) such that phosphorylation of high mannose containing recombinant Naglu protein by GNPT can take place inside the cells.

In some embodiments, the contacting of Naglu protein with GNPT may be conducted in the presence of a Naglu inhibitor, such as, but not limited to, 6AC-CAS (6-acetamido-6-deoxycastanospermine).

### Provided M6P-containing Recombinant Naglu Protein

Thus, the present invention provides M6P-containing recombinant Naglu protein. In some embodiments, M6P containing recombinant Naglu protein comprises capped-M6P, 1M6P (mono-M6P) and/or 2M6P (bis-phosphorylated M6P) containing glycans.

In some embodiments, M6P-containing recombinant Naglu protein provided by the present invention contains one and more M6P residues. For example, M6P-containing recombinant Naglu protein provided by the present invention contain at least 0.5, 1, 2, 3, 4, 5, 6, 7, 8, 9 or 10 M6P residues per protein.

In some embodiments, M6P-containing recombinant Naglu protein provided by the present invention may contain an increase in the number and/or level of total phosphorylated structures, compared to the naturally-occuring protein or protein not produced according to the present invention. For example, the increase in total M6P residues per protein compared to the naturally-occuring protein or protein not produced according to the present invention is by at least 10, 15, 20, 25, 30, 35, 40, 45, 50, 55, 60, 65, 70, 75, 80, 85, 90, 95, 96, 97, 98, 99 percent, 1-fold, 1.5-fold, 2-fold, 2.5-fold, 3-fold, 4-fold, or 5-fold.

In some embodiments, a recombinant Naglu protein produced according to the present invention may contain a lower level of bi- or multi-antennary structures compared to the naturally-occuring protein or protein not produced according to the present invention. In particular, a recombinant Naglu protein produced according to the present invention may have a higher level of A0 antennarity, which is indicative of M6P phosphorylation. In some embodiments, the increase in A0 antennarity compared to the naturally-occuring protein or protein not produced according to the present invention is by at least 10, 15, 20, 25, 30, 35, 40, 45, 50, 55, 60, 65, 70, 75, 80, 85, 90, 95, 96, 97, 98, 99 percent, 1-fold, 1.5-fold, 2-fold, 2.5-fold, 3-fold, 4-fold, or 5-fold.

In some embodiments, a recombinant Naglu protein produced according to the present invention may contain a bis-phosphorylated oligosaccharide which has higher binding affinity to the CIM6PR. In some embodiments, a suitable enzyme contains up to, on average, at least 20% bis-phosphorylated oligosaccharides per enzyme. In other embodiments, a suitable enzyme may contain about at least 10%, 15%, 18%, 20%, 25%, 30%, 35%, 40%, 45%, 50%, 55%, 60% bis-phosphorylated oligosaccharides per enzyme.

In some embodiments, the increased level in M6P phosphorylation of a recombinant Naglu protein produced according to the present invention may be tested using any of a variety of well-known assays, including binding assays to evaluate the level of CI-M6PR binding. In some embodiments, M6P-containing recombinant Naglu protein produced according to the present invention has an increase in the CI-M6PR binding by at least 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100-fold compared to the naturally-occuring Naglu protein or protein not produced according to the present invention.

In some embodiments, the level of M6P phosphorylation may be tested by monitoring binding of a therapeutic glycoprotein to the CI-M6PR using any of a variety of well-known *in vitro* binding assay such as, but not limited to, radiolabeled run on assay, radiolabeled binding assay, ELISA, Surface Plasmone Resonance and Isothermal Titration Calorimetry. In some embodiments, the level of M6P phosphorylation may be tested by monitoring binding of a therapeutic glycoprotein to the CI-M6PR by evaluating cellular uptake using methods such as, but not limited to, immunohistochemistry staining, fluorescent immunocytochemistry staining and confocal microscopy. In some embodiments, the level of M6P phosphorylation may be assayed using analytical techniques such as, but not limited to, glycosidase treatment, mass spectrometry and HPLC.

In some embodiments, the recombinant Naglu protein provided by the present invention is further characterized by high mannose containing glycans and/or sialic acid containing glycans. In some embodiments, the sialic acid containing glycans comprise one, two, three, and/or four sialic acid containing glycans.

### Pharmaceutical Compositions and Administration

The present disclosure further provides pharmaceutical compositions containing targeted therapeutics according to the present disclosure. Typically, suitable pharmaceutical compositions contain at least one pharmaceutically acceptable excipient and are formulated for administration to humans.

For example, pharmaceutical compositions provided herein may be provided in a sterile injectable form (e.g., a form that is suitable for subcutaneous, intravenous, or intrathecal injection). For example, pharmaceutical compositions can be provided in a liquid dosage form that is suitable for injection. Pharmaceutical compositions can be provided as powders (e.g., lyophilized and/or sterilized), optionally under vacuum, which are reconstituted with an aqueous diluent (e.g., water, buffer, salt solution, etc.) prior to injection. Pharmaceutical compositions can be diluted and/or reconstituted in water, sodium chloride solution, sodium acetate solution, benzyl alcohol solution, phosphate buffered saline, etc. Powder can be mixed gently with the aqueous diluent (e.g., not shaken).

Provided pharmaceutical compositions can comprise one or more pharmaceutically acceptable excipients (e.g., preservative, inert diluent, dispersing agent, surface active agent and/or emulsifier, buffering agent, etc.). Pharmaceutical compositions can comprise one or more preservatives. Pharmaceutical compositions can comprise no preservative.

Compositions of the pharmaceutical compositions described herein may be prepared by any method known or hereafter developed in the art of pharmacology. Such preparatory methods can include the step of bringing active ingredient into association with one or more excipients and/or one or more other accessory ingredients, and then, if necessary and/or desirable, shaping and/or packaging the product into a desired single- or multi-dose unit.

A pharmaceutical composition produced in accordance with the method of the present invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to a dose which would be administered to a subject and/or a convenient fraction of such a dose such as, for example, one-half or one-third of such a dose.

Relative amounts of active ingredient, pharmaceutically acceptable excipient, and/or any additional ingredients in a pharmaceutical composition produced in accordance with the method of the present invention may vary, depending upon the identity, size, and/or condition of the subject treated and/or depending upon the route by which the composition is to be administered. By way of example, the composition may comprise between 0.1% and 100% (w/w) active ingredient.

Pharmaceutical compositions produced in accordance with the method of the present invention may additionally comprise a pharmaceutically acceptable excipient, which, as used herein, may be or comprise solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro, (Lippincott, Williams & Wilkins, Baltimore, MD, 2006) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this disclosure.

Pharmaceutical compositions produced in accordance with the method of the present invention can be used for CNS delivery via various techniques and routes including, but not limited to, intraparenchymal, intracerebral, intraventricular cerebral (ICV), intrathecal (e.g., IT-Lumbar, IT-cisterna magna) administrations and any other techniques and routes for injection directly or indirectly to the CNS and/or CSF.

Pharmaceutical compositions produced in accordance with the method of the present invention can be used for intrathecal administration. As used herein, intrathecal administration (also referred to as intrathecal injection or intrathecal delivery) refers to an injection into the spinal canal (intrathecal space surrounding the spinal cord). Various formulations for intrathecal administration are described in WO/2011/163652.

A pharmaceutical composition containing a targeted therapeutics produced in accordance with the method of the present invention may be injected at any region surrounding the spinal canal. A pharmaceutical composition can contain a targeted therapeutics is injected into the lumbar area or the cisterna magna or intraventricularly into a cerebral ventricle space. As used herein, the term "lumbar region" or "lumbar area" refers to the area between the third and fourth lumbar (lower back) vertebrae and, more inclusively, the L2-S1 region of the spine. Typically, intrathecal injection via the lumbar region or lumber area is also referred to as "lumbar IT delivery" or "lumbar IT administration."

Various devices may be used for intrathecal delivery. A device for intrathecal administration can contain a fluid access port (e.g., injectable port); a hollow body (e.g., catheter) having a first flow orifice in fluid communication with the fluid access port and a second flow orifice configured for insertion into spinal cord; and a securing mechanism for securing the insertion of the hollow body in the spinal cord. As a non-limiting example, a suitable securing mechanism contains one or more nobs mounted on the surface of the hollow body and a sutured ring adjustable over the one or more nobs to prevent the hollow body (e.g., catheter) from slipping out of the spinal cord. The fluid access port can comprise a reservoir. The fluid access port can comprise a mechanical pump (e.g., an infusion pump). An implanted catheter can be connected to either a reservoir (e.g., for bolus delivery), or an infusion pump. The fluid access port may be implanted or external

Intrathecal administration may be performed by either lumbar puncture (i.e., slow bolus) or via a port-catheter delivery system (i.e., infusion or bolus). The catheter can be inserted between the laminae of the lumbar vertebrae and the tip is threaded up the thecal space to the desired level (generally L3-L4).

For injection, formulations produced in accordance with the method of the invention can be formulated in liquid solutions. In addition, the enzyme may be formulated in solid form and re-dissolved or suspended immediately prior to use. Lyophilized forms are also included. The injection can be, for example, in the form of a bolus injection or continuous infusion (e.g., using infusion pumps) of the enzyme.

### Treatment of San B

The M6P-containing Naglu produced in accordance with the method of the present invention may be used to effectively treat Sanfilippo Syndrome Type B. Sanfilippo Syndrome Type B, or Mucopolysaccharidosis III B (MPS III B). Naglu is localized to lysosomes and plays an important role in the catabolism of glycosaminoglycans (GAGs) heparan- and dermatan-sulfate. In the absence of enzyme, these substrates accumulate within cells, ultimately causing engorgement, followed by cellular death and tissue destruction. Due to the widespread expression of enzyme, multiple cell types and organ systems are affected in MPS III B patients.

A defining clinical feature of this disorder is central nervous system (CNS) degeneration, which results in cognitive impairment (e.g., decrease in IQ). Additionally, MRI scans of affected individuals have revealed white matter lesions, dilated perivascular spaces in the brain parenchyma, ganglia, corpus callosum, and brainstem; atrophy; and ventriculomegaly (Wang et al. Molecular Genetics and Metabolism, 2009). The disease typically manifests itself in the first years of life with organomegaly and skeletal abnormalities. Some affected individuals experience a progressive loss of cognitive function, with most affected individuals dying of disease-associated complications in their first or second decade.

Naglu compositions produced in accordance with the method of the present invention may be used to effectively treat individuals suffering from or susceptible to Sanfilippo Syndrome Type B. The terms, "treat" or "treatment," as used herein, refers to amelioration of one or more symptoms associated with the disease, prevention or delay of the onset of one or more symptoms of the disease, and/or lessening of the severity or frequency of one or more symptoms of the disease.

Treatment can refer to partially or complete alleviation, amelioration, relief, inhibition, delaying onset, reducing severity and/or incidence of neurological impairment in a Sanfilippo Syndrome Type B patient. As used herein, the term "neurological impairment" includes various symptoms associated with impairment of the central nervous system (e.g., the brain and spinal cord). Symptoms of neurological impairment may include, for example, e.g., cognitive impairment; white matter lesions; dilated perivascular spaces in the brain parenchyma, ganglia, corpus callosum, and/or brainstem; atrophy; and/or ventriculomegaly, among others.

The terms, "improve," "increase" or "reduce," as used herein, indicate values that are relative to a control. A suitable control can be a baseline measurement, such as a measurement in the same individual prior to initiation of the treatment described herein, or a measurement in a control individual (or multiple control individuals) in the absence of the treatment described herein. A "control individual" is an individual afflicted with a lysosomal storage disease (e.g., Sanfillipo Syndrome Type B), who is about the same age and/or gender as the individual suffering from the same lysosmal storage disease, who is being treated (to ensure that the stages of the disease in the treated individual and the control individual(s) are comparable).

The individual (also referred to as "patient" or "subject") being treated is an individual (fetus, infant, child, adolescent, or adult human) having a lysosomal storage disease or having the potential to develop a lysosmal storage disease. The lysosmal storage disease can be Sanfilippo Syndrome. The lysosomal storage disease can be Sanfilippo Syndrome Type B. The individual can have residual endogenous Naglu expression and/or activity, or no measurable activity. For example, the individual having Sanfilipo Syndrome Type B may have Naglu expression levels that are less than about 30-50%, less than about 25-30%, less than about 20-25%, less than about 15-20%, less than about 10-15%, less than about 5-10%, less than about 0.1-5% of normal Naglu expression levels.

The individual can be an individual who has been recently diagnosed with the disease. Typically, early treatment (treatment commencing as soon as possible after diagnosis) is important to minimize the effects of the disease and to maximize the benefits of treatment.

The invention will be more fully understood by reference to the following examples. They should not, however, be construed as limiting the scope of the invention.

### EXAMPLES

The following Examples are provided in support of certain methods of the present invention. For instance, the below examples and present invention demonstrate and/or suggest, among other things, that UDP-GlcNac, a phosphate donor for GNPT capable of transferring phosphate to mannose glycan on glycoprotein, could also be utilized by Naglu. In a PAD-HPLC experiment, in which rhNaglu and UDP-GlcNac are incubated together, the breakdown of UDP-GlcNac into the product GlcNac was observed. Also observed was the inhibition of Naglu activity by UDP-GlcNac. When added to an activity assay with a fluorescent substrate, UDP-GlcNac inhibited Naglu activity, suggesting competitive binding of UDP-GlcNac to Naglu on the active site. Data presented herein suggest the possibility that Naglu can bind to UPD-GlcNac at a lower binding affinity than GNPT, which difference of affinity could explain differences observed in the phosphorylation of Naglu between recombinant and endogenous cell lines. In recombinant cell lines, in which expression of Naglu is drastically increased, the binding of Naglu to UDP-GlcNac could deprive GNPT of, or block access of GNPT to, proper substrate, resulting in low GNPT activity and leading to a lack of phosphorylation of recombinant Naglu. In endogenous cell lines, where the expression of Naglu is at its minimum, UDP-GlcNac would be readily available to GNPT, therefore resulting in proper phosphorylation of endogenous Naglu.

### Example 1: Materials

*Recombinant Proteins* - Recombinant human Naglu (rhNaglu), Naglu-Kif, soluble GNPT (sGNPT) and soluble CI-M6PR (sCI-M6PR) were produced as follows. Recombinant human Naglu was purified from cell culture of a clone of human fibrosacroma HT1080 cells engineered to over-express rhNaglu. Naglu-Kif, a high-mannose-containing recombinant human Naglu, was purified from cell cultures of the same human fibrosacroma HT1080 cell clone wherein the growth medium was supplemented with 2mg/l Kifunensine. Soluble GNPT was a HPC4 tagged GNPT (SEQ ID NO.: 5) and was generated and purified according to published protocolls (Kudo and Canfield 2006). Soluble CI-M6PR was purified from cell cultures of a recombinant cell line that over-expresses sCI-M6PR.

Rabbit polyclonal anti-Naglu antibody (#2957) was generated by immunizing a rabbit with purified rhNaglu. Antibodies from the rabbit were protein G-purified prior to use.

### Example 2: Methods

*Naglu Substrate Analysis* - Recombinant human Naglu was co-incubated with uridine diphosphate N-acetylglucosamine (UDP-GlcNAc) or uridine diphosphate N-acetyl galactosamine (UDP-GalNAc). UDP-GlcNAc, GlcNAc (N-acetylglucosamine) and UDP-GalNAc were detected using PAD-HPLC (Pulsed Amperometric Detection- High Pressure Liquid Chromatography) that was modified after Chavaroche et al. 2011.

Recombinant human Naglu was dialyzed into Tris buffer (40 mM Tris pH 7.15, 100 mM NaCl, 4 mM MgCl₂, and 4 mM MnCl₂) and concentrated with VivaSpin® sample concentrators having a 30kDa cut-off (Viva Products, Inc,. Littleton, MA). The protein was then mixed with UDP-GlcNAc or UDP-GalNAc separately in Tris buffer and incubated at 37°C for 1 hour. After incubation, the samples were centrifuged for 5 minutes at 10,000 rpm, and the supernatants were loaded on a Dionex ICS-3000 Ion Chromatography System (Thermo Fisher Scientific, Inc., Sunnyvale, CA). Mobile phase A contained 100 mM NaOH, and Mobile Phase B contained 100 mM NaOH + 1M NaOAc. Chromatography gradients were set as follows: 0-5 min 100% A, 5-35 min 0-100% B, 35-40 min 100% B, 40-45 min 100-0% B, 45-48 min 100% A. The flow rate was set at 0.3 mL/min. UDP-GlcNAc and UDP-GalNAc eluted from the column at around 40.9 minutes, and GlcNAc eluted from the column at 10.7 minutes.

*Naglu Activity Assay with Various Inhibitors*: A 10 mM stock of Naglu activity substrate solution was prepared by dissolving 5 mg of substrate, i.e., 4-methylumbelliferyl-N-acetyl-α-D-glucosaminide, in 250 µl of 100% DMSO. Naglu activity assay reaction buffer (0.1 M sodium acetate, 0.5mg/ml BSA, pH 4.5) was then added to bring the volume up to 1318 µl. This 10 mM substrate stock solution was then diluted 10 fold with reaction buffer to make the final 1 mM substrate solution.

Samples and controls were pre-diluted in reaction buffer. 10 µl of each pre-diluted sample/control were applied in duplicate wells on a black clear bottom 96 well plate. 75 µl of 1 mM substrate solution containing various concentrations of inhibitors were added to each well. The plates were sealed with plate sealer and wrapped with aluminum foil. The plates were then placed in a Boekel Jitterbug Microplate Incubator Shaker (Cole-Parmer, Vernon Hills, IL) and shaken (setting at #1) at 37°C for 1 hour. 200 µl of Stop Buffer (0.2 M Glycine, pH 10.7) were then added to all of the wells and fluorescence emission (excitation/emission at 360 nm/460 nm, respectively) was measured using a SpectraMax® Plus M2 plate reader from Molecular Devices (Sunnyvale, CA).

*Analysis of Glycan Structure of rhNaglu* - (1) In-Gel Glycan Release and 2-AB Labeling - Protein was separated by SDS-PAGE. Gels were washed with ddH₂O and stained overnight in Gelcode Blue safe stain (Thermo Scientific, UK). Gels were destained with ddH₂O and target bands were excised with sterile scalpels. Glycans were released from gel-excised glycoprotein bands as previously described (Bigge et al., 1995; Guile et al., 1996; Royle et al., 2006), except for a few minor modifications. N-linked glycans were released from gel bands excised from polyacrylamide gels and labeled with 2-aminobenzamide (2-AB) as previously described (Royle et al., 2006). Following the labeling of glycans with 2-AB, excess label was removed using Phytip® columns (Phynexus, Inc., San Jose, CA) loaded with normal phase resin. Briefly, individual columns were conditioned by cycling 95% (v/v) acetonitrile with 20% (v/v) acetonitrile in three iterations. Glycans were reconstituted in 90% (v/v) acetonitrile and loaded onto individual Phytip® colums. Excess 2AB was washed off the colums using 95% (v/v) acetonitrile, and 2-AB labeled glycans were then eluted with 20% (v/v) acetonitrile. Excess acetonitrile was removed from the labeled glycans by vacuum evaporation and the resulting glycan samples were reconstituted in a predetermined volume of Milli-Q®-grade ddH₂O.
(2) Chromatography (HI-UPLC) - Labeled glycans were prepared as 25 µl aliquots in 60% (v/v) acetonitrile and separated on an Acquity UPLC H-Class chromatography system outfitted with a BEH glycan column (2.1 mm x 150 mm) containing 1.7 µm particles, both from Waters Corporation (Milford, MA), as previously described (Ahn et al., 2010). Separated glycan peaks were then analyzed using Empower 3 chromatography data software from the same company. Retention times corresponding to individual peaks were converted to GU values using dextran as an internal reference.
(3) Exoglycosidase Array Digestion of N-linked Glycans - Glycan deconvolution was performed using an array of exoglycosidase enzymes as previously described (Royle et al., 2006). Briefly, 5 µl of 2-AB labeled glycan material was dried down using vacuum evaporation, whereupon various combinations of exoglycosidases were added in the presence of 20 mM sodium acetate, pH 5.5. Each digestion reaction was incubated at 37°C overnight. Subsequently, enzyme material was removed with microfuge centrifugal filtration devices containing a 10 kDa MWCO. Digested glycan material was then prepared as 25 µl aliquots in 60% (v/v) acetonitrile and separated using the aforesaid chromatography system.
(4) Dephosphorylation of N-linked Glycans - Glycans were dephosphorylated using CIAP (Calf Intestinal Alkaline Phosphatase, Roche, Germany). Briefly, 5 µl of 2-AB labeled glycans were dried down using vacuum evaporation and incubated overnight at 37°C with 1 µl of CIAP (5 U µl/1) and 9 µl of 50 mM Tris-HCl 0.1 mM EDTA, pH 8.5. Enzyme material was then removed as described.

*Generation of Naglu-Kif, a high-mannose-containing recombinant Naglu -* Kifunensine is a potent inhibitor of alpha-mannosidase I in the ER (endoplasmic reticulum). Alpha-mannosidase I catalyzes the removal of terminal, non-reducing alpha-D-mannose residues from high mannose glycans; proteins produced in cells treated with kifunensine contain mostly Man9(GlcNAc)2 N-linked oligosaccharides.

*Glycodigestion and SDS-PAGE* - 2 µg of protein were digested with PNGase F (Peptide N-glycosidase F), Endo H (endoglycosidase H) (both New England Biolabs, Ipswitch, MA) overnight at 37°C in the buffers provided by the manufacturer. The digested proteins were mixed with SDS-PAGE sample buffer containing DTT, heated to 37°C for 15 minutes and then separated by SDS-PAGE. Afterwards, the gels were washed for 30 minutes in water and then stained for 1 hour with Gel Code Blue (Biorad, Hercules, CA).

*GNPT modification of Naglu-Kif*- Modification of Naglu-Kif (or rhNAglu as a control) by sGNPT was conducted in 100 µl reactions containing 30 µM UDP-GlcNAc (50 mM NaAc pH 6.5, 20 mM MnCl₂), appropriate amounts of sGNPT (50 mM NaAc pH 6.5, 150 mM NaCl, 20 mM MgCl₂), and 49.5 µg of Naglu-Kif (corresponding to about 5 µM) or rhNaglu in PBS. The final volume was made up by reaction buffer (50 mM NaAc pH 6.5, 20 mM MnCl₂). Reactions were incubated at 20°C for 24 hours.

*Plate-Based sCI-M6PR Binding Assay* - (1) Generation of sCI-M6PR (soluble CI-M6PR) - Full length M6PR was expressed in genetically modified tissue culture cells, and the secreted sCI-M6PR was purified from the tissue culture medium using an aminophenyl-M6P conjugated resin (Beljaars et al., 1999). N-terminal sequencing and LC/MS/MS analysis of the obtained protein confirmed the amino acid sequence of the sCI-M6PR, and MALDI analysis confirmed that its molecular mass was 279,492 Da.

(2) Binding Assays - 100 µl of sCI-M6PR(2.5 µg/ml) in DPBS were coated on MaxiSorp® Nunc-Immuno flatt-bottom 96-well plates (Thermo Fisher Scientific, Inc., Sunnyvale, CA). After incubation for 1 hourr at 37°C, the plates was washed with ELISA wash buffer (0.1% Tween-20 in DPBS). sCI-M6PR coated wells were blocked with 300 µl ELISA blocking buffer (DPBS, 2% BSA, 0.05% Tween 20) at 37°C for 1 hour. Appropriate dilutions of sGNPT-modified Naglu-Kif (and rhNaglu as control) were made in blocking buffer and added to the wells. After 1 hour incubation at 37°C, the plates were washed with ELISA wash buffer, and 100 µl of HRP-conjugated rabbit anti-Naglu polyclonal antibody #2957 diluted in blocking buffer were added. After 1 hour incubation at 37°C, the plates was washed again with ELISA wash buffer. The ELISA was developed using TMB peroxidase substrate solution (KPL, Inc., Gaithersburg, MA) by following the manufacturer's protocol.

*Mannose-6-Phosphate monosaccharide Quantitation* - The procedure for mannose-6-phosphate quantitation by HPLC was adapted from Zhou et al. 2002. 95 µl of sGNPT-treated Naglu-Kif were added to Eppendorf tubes and then 103 µl of 13 M trifluoroacetic acid (TFA) were added to a final concentration of 6.75 M. The mixture was then transferred to a 0.5 ml glass vial with a screw cap. Standards were prepared using mannose-6-phosphate disodium purchased from Sigma-Aldrich Corporation (St. Louis. MO). The standards were prepared in H₂O/TFA without heating in concentrations ranging from 50 to 1000 pM, and were then transferred to glass vials with screw caps. All samples were heated at 100°C for 1.5 hours in a heat block. After heating, the tubes were placed on ice for 5 minutes and then transferred to Eppendorf tubes. The samples were centrifuged at 14,000 rpm for 2 minutes and then dried in a SpeedVac® concentrator for ∼1.5 hours. 200 µl of Milli-Q® water were then added to each sample and the samples were again dried as before. Then, 100 µl of Milli-Q® water were added to each sample and the samples were filtered through a 0.45 µm Durapore® centrifugal filter unit (Millipore, Billerica, MA) at 14,000 rpm for 5 minutes. 20 microliters of each sample and standards were injected in a chromatography system with a borate trap column in line with a CarboPac PA 10 analytical column (Dionex ICS-3000 Ion Chromatography System (Thermo Fisher Scientific, Inc., Sunnyvale, CA)). An isocratic gradient was run with at 80% Eluent A (100 mM NaOH) and 20% Eluent B (1 M NaAc in 100 mM NaOH) for 15 minutes with a step up to 70% Eluent B for 5 minutes, then back to 20% Eluent B.

*PAD-HPLC Method for the Detection of Cap-M6P Containing Glycans* - To monitor the distribution of the glycan (oligosaccharide) chains on sGNPT-modified Naglu-Kif, a technique employing high pH anion exchange chromatography of the released and 2-AB labeled glycans was developed. Briefly, Naglu-Kif was heat denaturated at 100°C for 4 minutes and then subjected to treatment with peptide-N-Glycosidase F (PNGase F; 30 mU/3µl) for 20 ± 2 hours at 37°C to release the glycans from the protein. The next day, the released glycans were incubated with the fluorescent label 2-aminobenzamide (2-AB) for 4 hours at 55°C. Free label was removed by precipitation with acetone. The profile of the released glycans was then determined by High Performance Anion Exchange Chromatography with Fluorescent Detection (HPAEC-FLD). Briefly, samples were loaded on a Dionex CarboPac® PA-100 Analytical Columns equipped with a Dionex CarboPac PA-100® Guard Column (Dionex ICS-5000 with a FLD-3400RS detector (Thermo Fisher Scientific, Inc., Sunnyvale, CA)). The following conditions eluted the glycans in the order of increasing charge characteristics: The initial solvent of 3 mM sodium acetate/75 mM NaOH was run for 10 minutes, followed by a 1.6 mM/min sodium acetate gradient in 75 mM NaOH for 144 minutes at a flow rate of 0.25 ml/min and a 27°C column temperature.

To confirm that glycans contained Cap-M6P, samples were also treated with alkaline phosphatase and alpha-mannosidase after PNGase F digestion and subjected to the same HPLC regimen.

### Example 3: Hydrolysis of UDP-GIcNAc by Naglu

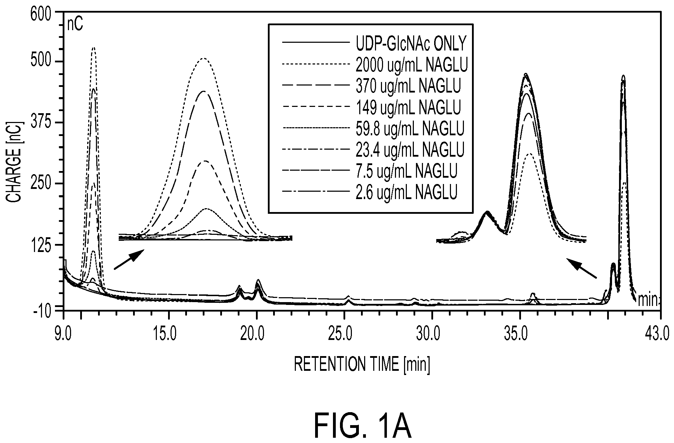

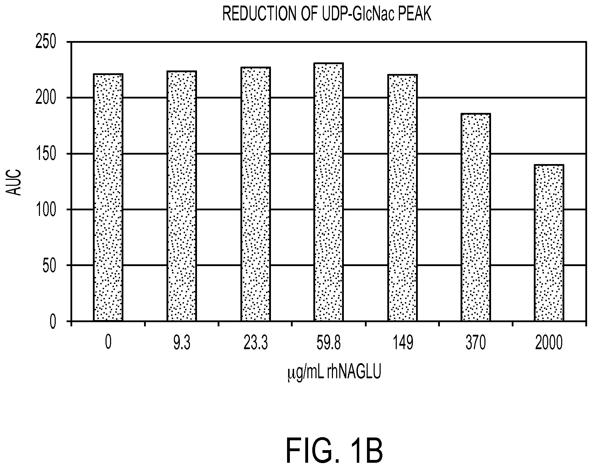

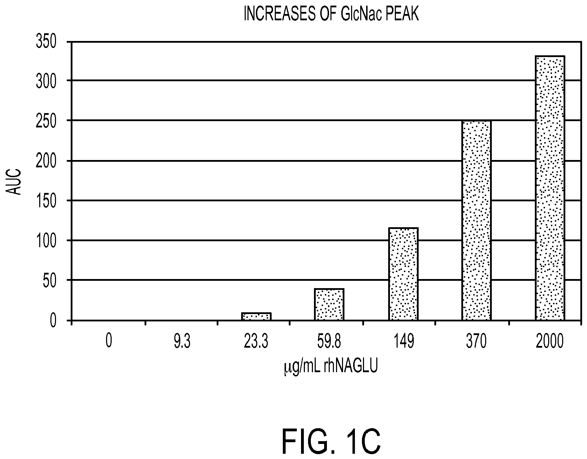

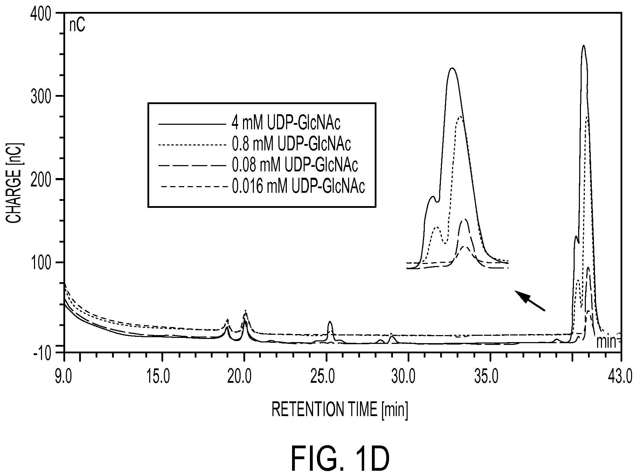

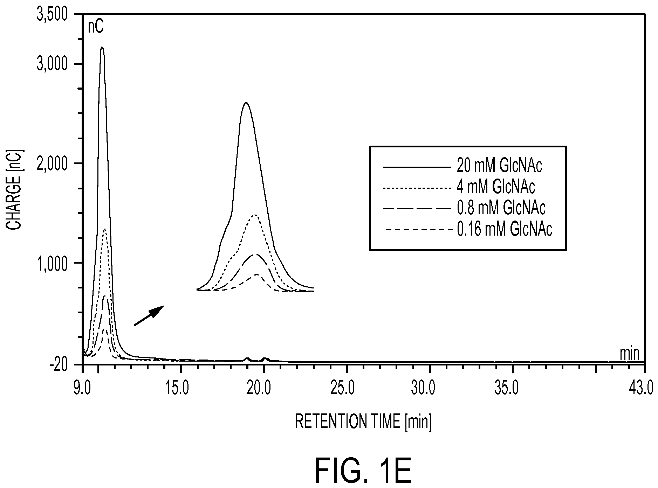

To demonstrate that Naglu could hydrolyze UDP-GlcNAc, UDP-GlcNAc (0.8 mM) was mixed with rhNaglu at concentrations of 2.6, 7.5, 23.4, 59.8, 149, 370 and 2,000 µg/ml in Tris buffer (40 mM Tris pH 7.15, 100 mM NaCl, 4 mM MgCl₂, and 4 mM MnCl₂) and incubated at 37°C for 1 hour. Samples were then subjected to centrifugation at 10,000 rpm, and the resulting supernatants were loaded on PA10 columns and detected by PAD-HPLC (Pulsed Amperometric Detection- High Pressure Liquid Chromatography) (Dionex ICS-3000 Ion Chromatography System (Thermo Fisher Scientific, Inc., Sunnyvale, CA)). The elution time of UDP-GlcNAc from the HPLC column was 40.9 minutes (FIG. 1A and FIG. 1D). The peak areas for the eluted UDP-GlcNAc were measured, and the reductions of the area under the curve (AUC) in response to increased concentrations of rhNaglu were determined (FIG. 1A). A new peak at 10.7 minutes was detected on the chromatograms of samples treated with concentrations of rhNaglu of 23.4 µg/ml and above. This peak correlated with GlcNAc and was not present in the original UDP-GlcNAc sample (FIG. 1A and FIG. 1E). The peak areas for the eluted GlcNAc were measured, and the increases of the AUC in response to increased concentrations of rhNaglu were determined (FIG. 1B). It appeared that under the experimental conditions tested, 23.4 µg/ml rhNaglu was the minimum concentration required for the detection of the hydrolysis of UDP-GlcNAc by rhNaglu (FIG. 1C).

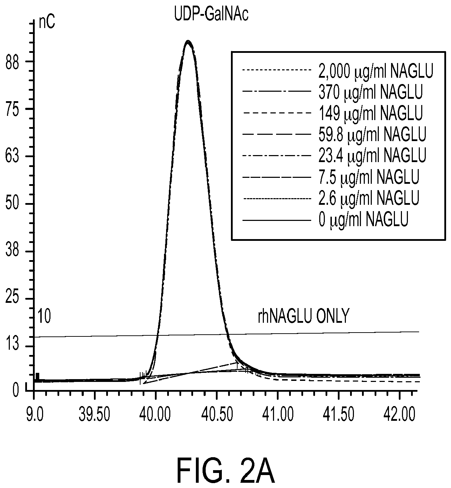

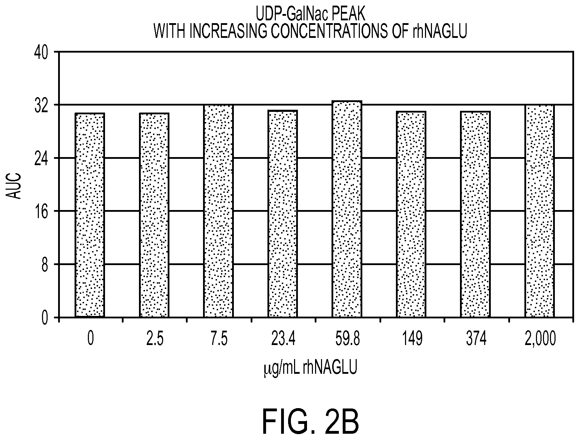

To further confirm that the hydrolysis of UDP-GlcNAc by rhNaglu was specific to UDP-GlcNAc, an epimer of UDP-GlcNAc, UDP-GalNAc (0.8 mM), was mixed with the same concentrations of rhNaglu and subjected to the same reaction conditions. The UDP-GalNAc reaction samples were examined by the same chromatography method as the UDP-GlcNAc reaction samples before. UDP-GalNAc eluted at the same place on the HPLC column as UDP-GlcNAc, i.e., at 40.9 minutes (FIG. 2A), which indicated that the two molecules have very similar charge characteristics. The peak areas for UDP-GalNAc were not reduced as the concentrations of rhNaglu increased, and no new peaks could be detected in the reactions (FIG. 2B), indicating that rhNaglu hydrolyses UDP-GlcNAc specifically, but not UDP-GalNAc.

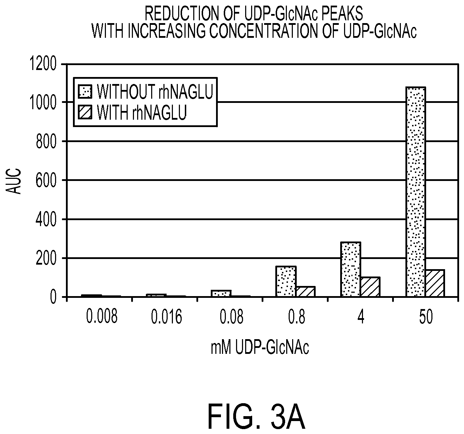

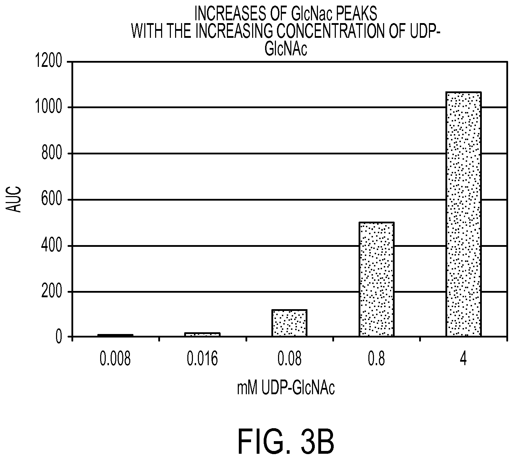

To further investigate the hydrolysis of UDP-GlcNAc by rhNaglu, increasing concentrations of UDP-GlcNAc (0.008, 0.016, 0.08, 0.8, 4.0 and 50 mM) were mixed with a constant concentration of rhNaglu (2.4 µg/ml) and incubated at 37°C for 1 hour. The AUC of the UDP-GlcNAc (FIG. 3A) and GlcNAc (FIG. 3B) peaks of each reaction were measured as before.

For each concentration of UDP-GlcNAc examined, a sample that did not contain rhNaglu was also examined as a control. The reduction of the UDP-GlcNAc substrate in the samples containing rhNaglu (FIG. 3A) and the formation of the reaction product GlcNAc in the samples containing rhNaglu (FIG. 3B) was apparent. The reductions of UDP-GlcNAc and the formation of GlcNAc were dependent on the initial concentration of UDP-GlcNAc in the reaction sample. Preliminary data derived from isothermal titration calorimetry (ITC) indicated that rhNaglu hydrolyses UDP-GlcNAc with a Kₘ that is in the milimolar range. This example demonstrates that rhNaglu recognizes and hydrolyzes UDP-GlcNAc.

### Example 4: UDP-GlcNAc Inhibition of rhNaglu Activity Toward 4MU-GlcNAc

To further confirm that rhNaglu could hydrolyze UDP-GlcNAc, we investigated whether UDP-GlcNAc inhibited the hydrolysis by rhNaglu of the fluorescent substrate 4MU-GlcNAc (4-methylumbelliferyl-N-acetyl-α-D-glucosaminide) in a Naglu activity assay. Briefly, UDP-GlcNAc, as well as GlcNAc, UDP-GalNAc and Glucosamine, were added to the Naglu activity assay at 0.0625, 0.25, 1, 4 and 8 mM concentrations. The fluorescence emitted from these samples was compared to control samples only including rhNaglu and 4MU-GlcNAc.

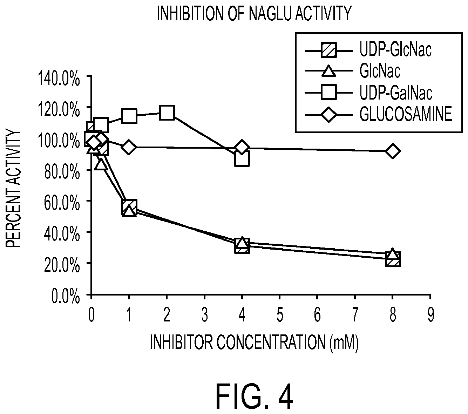

Inhibition of rhNaglu activity by UDP-GlcNAc and GlcNAc was observed (FIG. 4). Naglu activity was not inhibited by the epimer of UDP-GlcNAc, i.e., UDP-GalNAc, and glucosamine, which lacks the acetyl group at the 2-amine position. These results demonstrated that the hydrolysis of UDP-GlcNAc by Naglu was inhibiting Naglu's ability to hydrolyze 4MU-GlcNAc, and that the interaction of UDP-GlcNAc with rhNaglu occured at rhNaglu's active site.

The propensity of rhNaglu to hydrolyze UDP-GlcNAc explains why rhNaglu is not phosphorylated. sGNPT phosphorylates glycoproteins by transfering phospho-GlcNAc (p-GlcNAc) to their mannose residues. UDP-GlcNAc is a sugar precursor that serves as a substrate for sGNPT. In vivo, endogenous Naglu is expressed at a relatively low level so that it does not compete with sGNPT for substrate to an extent that reduces the ability of GNPT to phosphorylate Naglu. Endogenous Naglu is therefore phosphorylated by sGNPT.

The intracellular level of Naglu in cells over-expressing this protein can be several orders of magnitude higher than Naglu levels in endogenous cells. Therefore, in rhNaglu-overexpressing cells, rhNaglu competes with sGNPT for UDP-GlcNAc substrate and reduces the ability of sGNPT to phosphorylate rhNaglu. As shown elsewhere herein, this issue can be addressed by providing sGNPT in an amount greater than the amount of rhNaglu protein that is present, and/or by conducting the phosphorylation of rhNaglu in a test tube or bioreactor rather than in tissue culture cells.

### Example 5: Analysis of rhNaglu Glycan Structure

Another requirement for phosphorylation of rhNaglu by sGNPT was the availability of high-mannose-glycans that serve as the acceptor of the phospho-GlcNac group. Therefore, the structure of the glycans on rhNaglu was analyzed. Briefly, using the exoglycosidase array strategy previously described (Royle 2006), N-linked glycans were enzymatically removed from rhNaglu by PNGase F, labeled with 2-AB (2-aminobenzamide), which is a fluorescent dye for the tracking of glycans during chromatography, and then separated by HI-UHPLC (Hydrophobic Interaction Ultra-High Performance Liquid Chromatography) (FIG. 5). Exoglycosidase and alkaline phosphatase digestions were also performed on the glycans, and the reactions subsequently separated on the same type of HI-UHPLC columns. Retention times of the observed peaks were converted to GU (glucose unit) values and the relative peak areas were integrated.

Glycan peak shifts in GU values (retention time) due to exoglycosidase treatment were used to track glycan structural modification, and glycan peak shifts due to alkaline phosphatase treatment were used to identify M6P containing glycans. Using this approach, a total of 47 unique glycan structures were identified for rhNaglu (FIG. 5). The structures associated with the numbered peaks in FIG. 5 and the relative peak areas were calculated. A summary of the major characteristics, antennarity, sialylation, galactosylation, fucosylation and sialic acid linkage of glycan structures and the relative quantities is presented in FIG. 6.

Our analysis demonstrated that the glycans on rhNaglu are primarily bi- (33% (A2)) and tetra-antennary structures (45% (A4)) (FIG. 6A). No M6P-containing glycan structures or high mannose containing glycans (A0) were detected on rhNaglu (FIG. 6A). To put this value in context, the glycan structures of another recombinant lysosomal enzyme, rhArylsulfatase A, produced in the same cell type as rhNaglu was also evaluated, and significant amounts of high-mannose-glycans were found (40%(A0)). The extent of antennarity of the glycans was determined by adding peak areas of structures containing either no (A0), one (A1), two (A2), three (A3) or four (A4) GlcNAcs (in addition to the two proximal ones). A0 represented the high-mannose species, which was not present (0%) on rhNaglu (FIG. 6A). The extent of terminal sialylation (no (S0), one (S1), two (S2), three (S3), or four (S4) terminal sialic acids) (Figure 6B), and the presence of galactose units on GlcNAcs (no (G0), one (G1), two (G2), three (G3) or four (G4) galactose units) (FIG. 6C) was determined as well. The presence (F1) or absence (F0) of core fucosylation was also ascertained (FIG. 6D). The abundance of different sialic acid linkages was determined for the sialiated species (FIG. 6E). For the graph in FIG. 6E, only glycans containing terminal sialyl groups (i.e., which were S1, S2 and S3 on graph B) were taken into account. Therefore, the total amount of glycans on graph E was 56% and not 100%. This example demonstrates that in recombinant cell expression systems, rhNaglu does not carry high mannose glycans.

### Example 6: Generation of rhNaglu Carrying High Mannose Glycans, Naglu-Kif

The complete lack of high mannose glycan on rhNaglu was unexpected, and made it difficult to phosphorylate rhNaglu with GNPT in vivo or in vitro. To overcome this obstacle, rhNaglu containing high mannose glycan was generated in tissue culture cells grown in the presence of Kifunensine, which is an inhibitor of endoplasmic (ER) alpha-mannosidase I. The rhNaglu purified from these cultures contained high mannose glycans and was denoted Naglu-Kif.

During natural glycan processing in vivo, ER alpha-mannosidase I catalyzes the removal of terminal, non-reducing alpha-D-mannose on the Man9(GlcNAc)2 structures of N-glycan precursors. As the glycosylated protein enters the trans-Golgi, other mannosidase(s) often remove additional terminal mannoses from N-linked oligosaccharides, which results in Man8(GlcNAc)2, Man7(GlcNAc)2, Man6(GlcNAc)2 and Man5(GlcNA)c2 structures on N-linked oligosaccharides. Since mannose removal in vivo precedes the addition of GlcNAc for N-glycan branching and the subsequent addition of galactose and sialic acid residues for the generation of complex glycans, proteins produced in cells treated with Kifunensine contain predominately high mannose glycans and little or no complex N-linked glycans. The inhibition of ER alpha-mannosidase I by Kifunensine predominantly results in N-linked oligosaccharides with Man9(GlcNAc)2 structures.

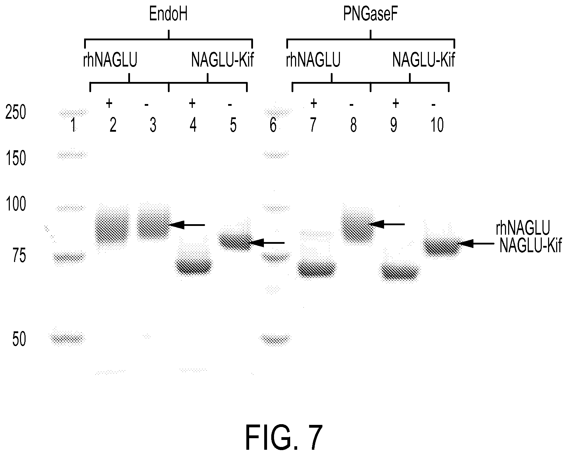

The difference between rhNaglu and the high mannose containing Naglu-Kif in terms of their N-linked glycan structure was demonstrated by Endo H and PNGase F digestion and SDS-PAGE analysis (FIG. 7). EndoH (Endoglycosidase H) removes high mannose and some hybrid glycans from N-linked glycoproteins, and PNGase F (Peptide -N-Glycosidase F) removes high mannose, hybrid, and complex oligosaccharides from glycoproteins. The removal of N-glycans from glycoproteins by either Endo H or PNGase F reduces the molecular weight of the treated glycoproteins and therefore inreases the speed with which they migrate in SDS-PAGE gels.

2 µg each of rhNaglu and Naglu-Kif were separately treated with Endo H and PNGase F, and then subjected to SDS-PAGE and Coomassie Blue statining (FIG. 7). The samples were distribed on the gel as follows. Lane 1 and 6: Molecular weight markers; lanes 2 and 3: rhNaglu with (+) or without (-) Endo H digestion; lanes 4 and 5: Naglu-Kif with or without Endo H digestion; lanes 7 and 8: rhNaglu with or without PNGase F digestion; lanes 9 and 10: Naglu-Kif with or without PNGase F digestion. The undigested rhNaglu appeared as a dispersed band compared to undigested Naglu-Kif (lane 3 and 8 vs. lane 5 and 10), a reflection of the complexity and heterogeneity of the N-glycans on rhNaglu. After Endo H digestion, the migration of rhNaglu on the gel was barely different from the undigested protein (lane 2 vs. lane 3), an indication of the lack of high mannose glycans on rhNaglu. Naglu-Kif migrated significantly faster after Endo H digestion (lane 4 vs. lane 5), a sign that the predominate content of glycans on Naglu-Kif were high mannose glycans. Also, it appeared that the migration of Naglu-Kif after Endo H digestion was very similar to its migration after PNGase F digestion (lane 7 vs. lane 9), another indication that most of the glycans on Naglu-Kif were high mannose glycans.

This example demonstrates that culturing recombinant human Naglu in the prescence of Kifunensine, an alpha mannosidase I inhibitor, leads to the production of high mannose-containing rhNaglu.

### Example 7: sGNPT phosphorylation of Naglu-Kif

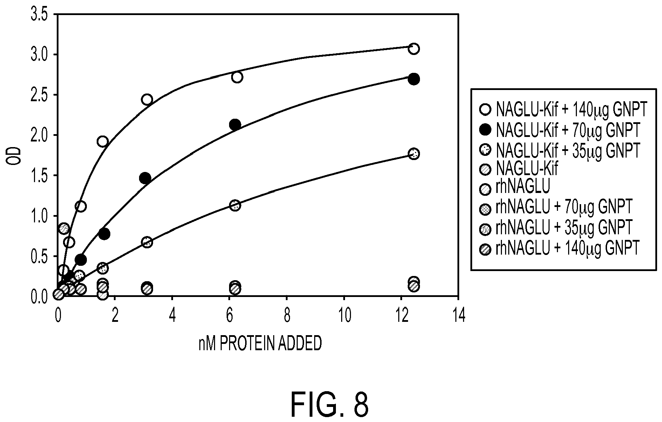

For the in vitro phosphorylation of Naglu-Kif (or rhNaglu as a control), the protein was mixed with increasing concentrations of sGNPT and incubated under the condition considered optimal for sGNPT activity (Kudo et al., 2006, Qian et al., 2010) (FIG. 8).

The results of the in vitro phosphorylation reactions were evaluated by three methods to directly or indirectly quantitate the M6P content of Naglu-Kif, i.e., a CI-M6PR binding assay, a M6P monosaccharide quantitation assay and a glycan profile analysis.

*CI-M6PR Binding Assay* - sGNPT transfers the phospho-GlcNAc group from UDP-GlcNAc to mannose sugar, and the resulting mannose-6-phosphate-GlcNAc is called the Capped-M6P. During the normal intracellular glycosylation process, the decapping enzyme (N-acetylglucosamine 1-phosphodiester α-N-acetyglucosaminidase) removes the GlcNAc from the Capped-M6P, to generate M6P. But without decapping, the Capped-M6P can also bind to CI-M6PR. Capped-M6P binds to domain 5 of CI-M6PR rather than to domain 3 or 9, as M6P does (Bohnsack et al. 2009). The amount of Capped-M6P on Naglu-Kif can be indirectly evaluated using a CI-M6PR plate binding assay. In this binding assay the affinity with which Naglu-Kif bound to CI-M6PR increased in proportion to the amount of sGNPT used (35 µg, 70 µg and 140 µg (correspondiong to about 4.2 µM) (green, red and empty circle in FIG. 8, respectively)) to modify Naglu-Kif and thus the extent of Capped-M6P found on Naglu-Kif (FIG. 8). The K_{D} values extrapolated from the binding assay data were 15.31 nM, 5.92 nM and 1.48 nM respectively. These binding reactions were repeated in two different buffer systems, i.e., in a sodium acetate buffer (40 mM NaAc pH 6.5, 10 mM MnCl₂, 10 mM MgCl₂) and in a Tris buffer (50 mM Tris pH 7.4, 10 mM MnCl₂, 10 mM MgCl₂), which gave similar results. Contrary to Naglu-Kif, rhNaglu was not phosphorylated by sGNPT under the same conditions due to the lack of high-mannose glycans. Accordingly, rhNaglu did not bind to CI-M6PR (FIG. 8).

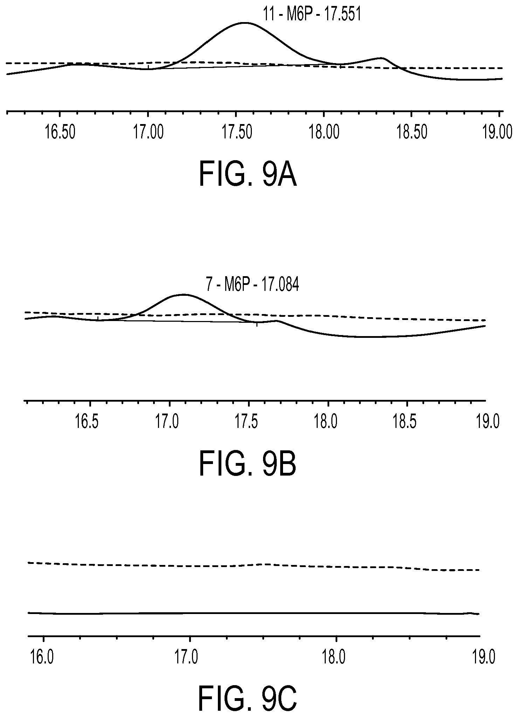

*M6P Monosaccharide Quantitation Assay* - To further confirm the existence of M6P on the phosphorylated Naglu-Kif, a monosaccharide M6P quantitation assay was performed using PAD-HPLC. This method used acid hydrolysis to break down the sugar molecules from all the glycans on the analyzed glycoproteins. Capped-M6P, under the acidic conditions of this assay, released GlcNAc, and the remaining M6P could be detected in this assay. M6P disodium salt was used to identify the peak of the M6P liberated from Naglu-Kif and for the generation of a standard curve allowing the determination of the amount of M6P presented on phosphorylated Naglu-Kif. M6P monosaccharide was quantified by PAD-HPLC using samples derived from Naglu-Kif treated with 140 ug (FIG. 9A), 70 ug (FIG. 9B) and 35 ug (FIG. 9C) of sGNPT per reaction. Parallel control experiments were conducted in which sGNPT was omitted (black line in FIG. 9). The AUC of the peak derived form the M6P liberated from Naglu-Kif increased in proportion to the sGNPT concentration used to phosphorylate Naglu-Kif (FIG. 9). The M6P content in samples derived from the reaction in which 35ug of sGNPT were used to treat Naglu-Kif was below the detection threshold (FIG. 9C). As an additional control, sGNPT alone was subjected to acid hydrolysis; no PAD-HPLC peak resulted from this experiment. The molar ratio of M6P over Naglu-Kif protein in various conditions was also determined and is presented in Table 4 below. Both the use of sodium acetate or Tris as reaction buffers resulted in robust phosphorylation of Naglu-Kif by sGNPT in vitro. The highest molar ratio of 1.45 pmol M6P/pmol protein was achieved where Naglu-Kif was phosphorylated with 70 µg sGNPT in Tris buffer.

**Table 4. The molar ratio of M6P/Naglu-Kif under various conditions is shown. The amount of M6P resulting from the phosphorylation of Naglu-Kif by sGNPT was calculated based on the AUC of the M6P peak on the PAD-HPLC chromatograms and by reference to standard curve generated from M6P disodium salt.**

| **Reaction Buffer** | **Sample** | **pmol M6P/pmol Naglu Kif** |
|---|---|---|
| 50 mM Sodium Acetate pH 6.5, 20 mM MnCl2 | +140 ug GNPT | 0.64 |
| | +70 ug GNPT | 0.12 |
| | +35 ug GNPT | N/D |
| 50 mM Tris pH 7.4, 10 mM MnC2, 10 mm MgCl2 | +70 ug GNPT | 1.45 |

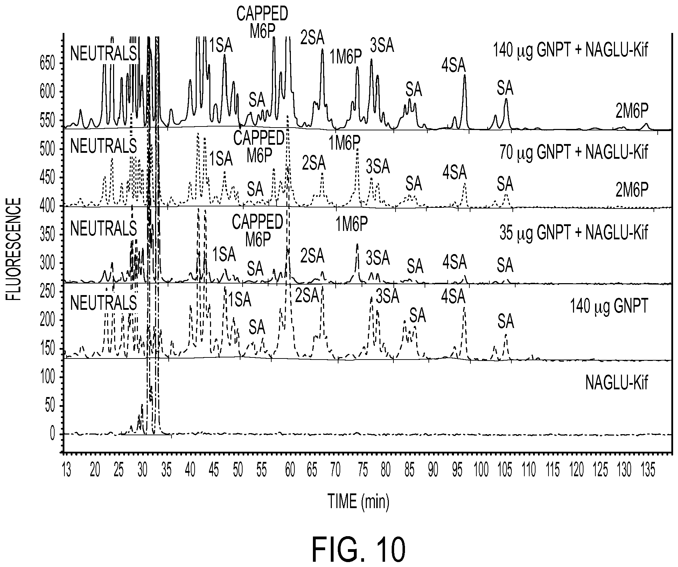

*Glycan Profile Analysis* - A more sensitive glycan profile analysis was performed to further confirm the existence of Capped-M6P glycans on Naglu-Kif after phosphorylation by sGNPT (FIG. 10). N-glycans from Naglu-Kif were released by treatment with PNGase F, labeled with the fluorescent dye 2-AB, and separated by HPLC according to the charge presented by the various sugars they contained, such as sialic acid or M6P.

Peaks were identified as representing neutral glycans (such as high mannose containing glycans), as representing glycans containing, one, two, three and four sialic acid residues (1SA, 2SA, 3SA and 4SA, respectively), as representing sialylated species with unindentified number of sialic acid (SA), as representing the Capped-M6P containing glycans, and as representing one or two M6P containing glycans (1M6P or 2M6P, respectively) (FIG. 10).

The glycan species that eluted earliest from the HPLC column were neutral glycans such as high mannose glycans, followed by glycans carrying one, two, three and four sialyl-residues (these glycan modifications are abbreviated herein as 1SA, 2SA, 3SA and 4SA, respectively). Glycans containing Capped-M6P eluted between 1SA-type and 2SA-type glycans. Glycans containing one terminal M6P (1M6P) eluted between 2SA-type and 3SA-type glycans. Glycans containing two terminal M6P groups (2M6P) had the highest charges of all the glycan species analyzed and therefore eluted from the HPLC column last (FIG. 10).

Capped-M6P glycan was found on all three samples of Naglu-Kif that were phosphorylated by sGNPT (FIG. 10). The peak area for the Capped-M6P glycans increased as sGNPT in the phosphorylation reaction increased from 35 µg to 70 µg and to 140 µg. Surprisingly, 1M6P and 2M6P containing species were also observed. The peak areas of these glycans also increased as the amount of sGNPT increased in the reactions, and these glycans were clearly not present in the sGNPT-only or Naglu-Kif-only samples. sGNPT transfers phospho-GlcNAc to mannose residues. Normally, the GlcNAc group is released and the M6P-groups exposed only after the decapping reaction by the decapping enzyme. Without wishing to be held to any particular theory in this regard, the applcants speculate that Naglu may also react with Capped-M6P to release the GlcNAc group and expose M6P. It is not likely that our experimental manipulations of the glycans per se favored the release of the GlcNAc group from the Capped-M6P because, for example, both the sGNPT reaction and glycan profile analysis were performed under neutral conditions.

The sialylted glycan species were observed in the sGNPT-only sample, and all the Naglu-Kif samples that contained sGNPT, but not in the Naglu-Kif sample without sGNPT, indicating that the sialylated species were contributed by the sGNPT that was in the phosphorylation reaction.

This example demonstrates the prescence of 1M6P or 2M6P on Naglu-Kif in addition to the Capped-M6P. Furthermore, the example also demonstrates that phosphorylation of Naglu-Kif by sGNPT occurs in a concentration dependent manner.

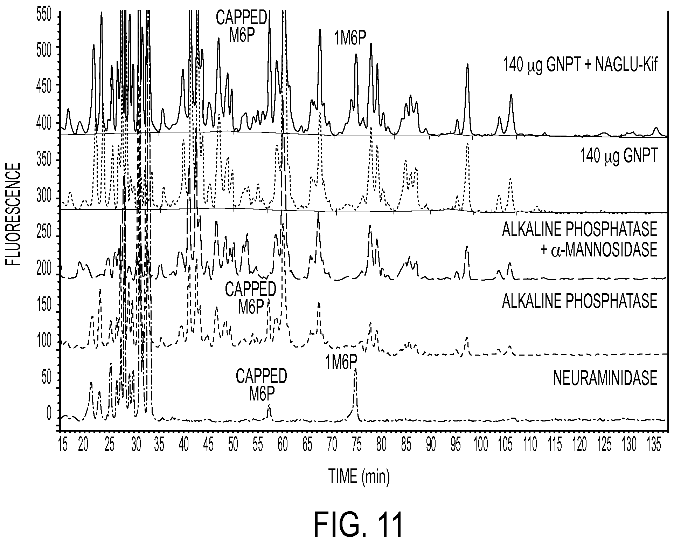

To confirm that glycans contained Cap-M6P, samples were also treated with neurominidase, alkaline phosphatase and alpha-mannosidase after PNGase F digestion and subjected to the same HPLC regimen as before (FIG. 11).

The glycan profile of Naglu-Kif phosphorylated by 140 µg of sGNPT shows the presence of neutral and sialiated glycans (1SA, 2SA, 3SA and 4SA), as well as those carrying Capped-M6P and 1M6P residues. The glycan profile of sGNPT alone shows the presence of primarily sialiated glycans; neither Capped-M6P nor 1M6P glycans were present. The treatment of the phosphorylation reaction with both alkaline phosphatase and alpha-mannosidase resulted in the disappearance of the peaks representing glycans carrying Capped-M6P and 1M6P groups from the chromatogram. The treatment of the phosphorylation reaction with alkaline phosphatase alone resulted in the disappearance of the peak representing glycans carrying 1M6P, but not of the peak representing glycans carrying Capped-M6P (the glycans from the 70 µg sGNPT + Naglu-Kif sample were used for alkaline phosphatase treatment). The treatment of the phosphorylation reaction with neuraminidase resulted in the dispappearance of the peaks representing sialiated glycans, and a significant increases of the peaks representing neutral glycans. The peaks representing glycans carrying Capped-M6P and 1M6P groups were still present (the glycans from the 35 µg sGNPT+Naglu-Kif sample was used for the neuraminidase treatment) (FIG. 11).

### Example 8: Phosphorylation of Naglu in Recombinant Cell lines

The present example addresses whether inactive Naglu would be properly phosphorylated in a recombinant cell line. Inactive Naglu of the present example is produced by introducing mutations of Naglu that reduce or eliminate one or more enzyme activities, such as mutations at the active site. Inactive rhNaglu is expressed in a mammalian cell line. The presence of phosphorylated glycans on the inactive rhNaglu is quantified or qualified.

### Example 9: Phosphorylation of Naglu in a Cell Line that Expresses a High Level of GNPT

The present example addresses whether a recombinant cell line that expresses a significantly high level of GNPT allows for proper phosphorylation of rhNaglu. In the present example rhNaglu and GNPT are co-expressed in a mammalian cell line. Screening is carried out to determine a GNPT level high enough to yield properly phosphorylated rhNaglu. Degree of GNPT overexpression is considered, as is potential cytotoxicity arising from GNPT overexpression in a recombinant system.

### REFERENCES

Ahn J1, Bones J, Yu YQ, Rudd PM, Gilar M. Separation of 2-aminobenzamide labeled glycans using hydrophilic interaction chromatography columns packed with 1.7 microm sorbent. J Chromatogr B Analyt Technol Biomed Life Sci. 2010 Feb 1;878(3-4):403-8
Anais A.E. Chavaroche, Lambertus A.M. van den Broek, Jan Springer, Carmen Boeriu and Gerrit Eggink. Analysis of the Polymerization initiation and activity of pasteurella multocida Heparoson Synthase PmHS2, an enzyme with glycosylatranferase and UDP-sugar Hydrolase Activity. The Journal of Biological Chemistry VoL. 286, No. 3, pp.1777-1785 Jan 21, 2011.
Bao M, Elmendorf BJ, J. Booth L, Drake RR, and Canfield WM. Bovine UDP-N-acetylglucosamine:Lysosomal-enzyme N-Acetylglucosamine-1-phosphotransferase. THE JOURNAL OF BIOLOGICAL CHEMISTRY, Vol. 271, No. 49, Issue of December 6, pp. 31446-31451, 1996
Beljaars L, Molema G, Weert B, Bonnema H, Oligna P, Groothuis G MM, Meijer DKF, Poelstra K. Albumin Modified With Mannose 6-Phosphate: A Potential Carrier for Selective Delivery of Antifibrotic Drugs to Rat and Human Hepatic Stellate Cells. HEPATOLOGY Vol. 29, No. 5, 1999
Bigge JC1, Patel TP, Bruce JA, Goulding PN, Charles SM, Parekh RB. Nonselective and efficient fluorescent labeling of glycans using 2-amino benzamide and anthranilic acid. Anal Biochem. 1995 Sep 20;230(2):229-38
Bohnsack RN, Song X, Olson LJ, Kudo M, Gotschall RR, Canfield WM, Cummings RD, Smith DF, and Dahms NM. Cation-independent Mannose 6-Phosphate Receptor A COMPOSITE OF DISTINCT PHOSPHOMANNOSYL BINDING SITES. THE JOURNAL OF BIOLOGICAL CHEMISTRY VOL. 284, NO. 50, pp. 35215-35226, December 11, 2009
Di Natale P, Salvatore D, Daniele A, Bonatti S. Biosythesis of Alpha-N-Acetylglucosaminidase in Cultured Human Kidney Carcinoma Cells. Enzyme 33, 75-83 (1985)
Guile GR1, Rudd PM, Wing DR, Prime SB, Dwek RA. A rapid high-resolution high-performance liquid chromatographic method for separating glycan mixtures and analyzing oligosaccharide profiles. Anal Biochem. 1996 Sep 5;240(2):210-26
Kudo M, and Canfield WM. Structural requirements for efficient processing and activation of recombinant human UDP-N-acetylglucosamine: Lysosomal-enzyme-N-acetylglucosamine-1-phosphotransferase. The Journal of Biological Chemistry Vol 281, No. 17, ppl 1761-11768 April 28, 2006
Qian Y, Lee I, Lee WS, Qian M, Kudo M, Canfield WM, Lobel P and Kornfeld S. Functions of the α,β, and γ Subunits of UDP-GlcNAc: Lysosomal Enzyme N-Acetylglucosamine-1-phosphotransferase. THE JOURNAL OF BIOLOGICAL CHEMISTRY VOL. 285, NO. 5, pp. 3360-3370, January 29, 2010
Reitman ML, Lang L, and Kornfeld S. UDP-N-Acetylglucosamine: Lysosomal enzyme N-Acetylglucosamine-1-phosphotransferase. Methods in enzymology, Vol. 107 163-172
Royle, L., C.M. Radcliffe, R.A. Dwek, and P.M. Rudd, Detailed structural analysis ofN-glycans released from glycoproteins in SDS-PAGE gel bands using HPLC combined with exoglycosidase array digestions. Methods Mol Biol, 2006. 347: p. 125-43.
Ullrich K, Basner R, Gieselmann V and Von Figura K. Recognition of Human Urine α-N-Acetylglucosaminidase by Rat Hepatocytes. Biochem. J. (1979) 180, 413-419
Ullrich K, Mersmann G, Weber E, Von Figura K. Evidence for Lysosomal Enzyme Recognition by Human Fibroblasts via a Phosphorylated Carbohydrate Moiety. Biochem. J. (1978) 170, 643-650
Von Figura K and Klein U. Isolation and Characterization of Phosphorylated Oligosaccharides from α-N-Acetylglucosaminidase that Are Recognized by Cell-Surface Receptors. Eur. J. Biochem. 94, 347-354 (1979)
Von Figura K and Kresse H. Quantitative Aspects of Pinocytosis and the Intracellular Fate of N-acetyl-α-D-glycosaminidase in Sanfilippo B Fibroblasts. The Journal of Clinical Investigation Volume 53 January 1974. 85-90
Yogalingam G, Weber B, Meehan J, RogerJ s, Hopwood JJ. Mucopolysaccharidosis type IIIB: charactereisation and expression of the wild-type and mutant recomb inant α-N-acetylglucosaminidase and relationship with Sanfilippo phenotype in an attenuated patient. Biochimica et Biophysica Aceta 1502 (2000) 415-425
Zhao KW and Neufeld EF. Purification and Characterization of Recombinant human α-N-acetylglucosaminidase secreted by Chinese Hamster Ovary Cells. Protein Expression and Purification 19, 202-211 (2000)
Zhou Q, Kyazike J, Edmunds T, and Higgins E, Mannose 6-Phosphate Quantitation in Glycoproteins Using High-pH Anion-Exchange Chromatography with Pulsed Amperometric Detection. Analytical Biochemistry 306, 163-170 (2002)

## Claims

1. A method of producing mannose-6-phosphate (M6P)-containing alpha-N-acetylglucosaminidase (Naglu), comprising the steps of:
providing a high mannose containing Naglu protein which has been generated from mammalian or non-mammalian cells using recombinant DNA technology; and
contacting the high mannose containing Naglu protein with N-acetylglucosamine-1-phosphotransferase (GNPT) under conditions that permit phosphorylation of one or more mannose residues on the Naglu protein, wherein conditions that permit phosphorylation comprise a greater than 1:1 GNPT to Naglu molar ratio such that GNPT is in excess of Naglu, thereby generating M6P-containing Naglu.

2. The method of claim 1, wherein the high mannose containing Naglu protein comprises Man9(GlcNAc)2, Man8(GlcNAc)2, Man7(GlcNAc)2, Man6(GlcNAc)2, and/or Man5(GlcNAc)2 N-linked oligosaccharides.

3. The method of claim 1 or 2, wherein the high mannose containing Naglu protein comprises Man9(GlcNAc)2 N-linked oligosaccharides.

4. The method of any one of the preceding claims, wherein the high mannose containing Naglu protein is produced from cells cultured in the presence of an alpha-mannosidase I inhibitor.

5. The method of claim 4, wherein the alpha-mannosidase I inhibitor is selected from the group consisting of deoxymannojirimycin, kifunensine, D-mannonolactam amidrazone, N-butyl-dexoymannojirimycin, and combinations thereof.

6. The method of claim 4 or 5, wherein the alpha-mannosidase I inhibitor is kifunensine.

7. The method of any one of claims 4-6, wherein the cells are mammalian cells.

8. The method of any one of claims 4-7, wherein the cells are human or CHO cells.

9. The method of any one of claims 4-8, wherein the method further comprises a step of purifying the high mannose containing Naglu protein from the cells.

10. The method of any one of the preceding claims, wherein contacting the high mannose containing Naglu protein with the GNPT is carried out in vitro.

11. The method of any one of the preceding claims, wherein contacting the high mannose containing Naglu protein with the GNPT is carried out in a reaction vessel.

12. The method of any one of the preceding claims, wherein the conditions that permit phosphorylation further comprise:
i) incubating the high mannose containing Naglu protein with the GNPT in the presence of UDP-GlcNac; and/or
ii) incubating the high mannose containing Naglu protein with the GNPT at about 20°C.

13. The method of any one of the preceding claims, wherein the method further comprises a step of incubating the M6P-containing Naglu with N-acetylglucosamine 1-phosphodiester α-N-acetylglucosaminidase.

14. The method of any one of the preceding claims, wherein the Naglu protein comprises an amino acid sequence of SEQ ID NO: 1.

15. The method of any one of the preceding claims, wherein the Naglu protein is not fused or conjugated to a peptide.

## Patentansprüche

1. Verfahren zur Herstellung von Mannose-6-phosphat (M6P) enthaltender alpha-N-Acetylglucosaminidase (Naglu), umfassend die Schritte:
Bereitstellen eines mannosereichen Naglu-Proteins, das aus Säuger- oder Nicht-Säugerzellen unter Verwendung rekombinanter DNA-Technologie erzeugt wurde; und
Inkontaktbringen des mannosereichen Naglu-Proteins mit N-Acetylglucosamin-1-phosphotransferase (GNPT) unter Bedingungen, die eine Phosphorylierung eines oder mehrerer Mannosereste auf dem Naglu-Protein ermöglichen, wobei Bedingungen, die eine Phosphorylierung ermöglichen, ein Molverhältnis von mehr als 1:1 GNPT zu Naglu umfassen, sodass die Menge an GNPT über der an Naglu liegt, wodurch M6P-haltiges Naglu erzeugt wird.

2. Verfahren nach Anspruch 1, wobei das mannosereiche Naglu-Protein Man9(GlcNAc)2, Man8(GlcNAc)2, Man7(GlcNAc)2, Man6(GlcNAc)2 und/oder Man5(GlcNAc)2 N-gebundene Oligosaccharide umfasst.

3. Verfahren nach Anspruch 1 oder 2, wobei das mannosereiche Naglu-Protein Man9(GlcNAc)2 N-gebundene Oligosaccharide umfasst.

4. Verfahren nach einem der vorstehenden Ansprüche, wobei das mannosereiche Naglu-Protein aus Zellen hergestellt wird, die in Gegenwart eines Alpha-Mannosidase-I-Inhibitors kultiviert wurden.

5. Verfahren nach Anspruch 4, wobei der Alpha-Mannosidase-I-Inhibitor ausgewählt ist aus der Gruppe bestehend aus Desoxymannojirimycin, Kifunensin, D-Mannonolactamamidrazon, N-Butyl-Desoxymannojirimycin und Kombinationen davon.

6. Verfahren nach Anspruch 4 oder 5, wobei es sich bei dem Alpha-Mannosidase-I-Inhibitor um Kifunensin handelt.

7. Verfahren nach einem der Ansprüche 4 bis 6, wobei es sich bei den Zellen um Säugerzellen handelt.

8. Verfahren nach einem der Ansprüche 4 bis 7, wobei es sich bei den Zellen um menschliche oder CHO-Zellen handelt.

9. Verfahren nach einem der Ansprüche 4 bis 8, wobei das Verfahren ferner einen Schritt der Reinigung des mannosereichen Naglu-Proteins aus den Zellen umfasst.

10. Verfahren nach einem der vorstehenden Ansprüche, wobei das Inkontaktbringen des mannosereichen Naglu-Proteins mit dem GNPT in vitro durchgeführt wird.

11. Verfahren nach einem der vorstehenden Ansprüche, wobei das Inkontaktbringen des mannosereichen Naglu-Proteins mit dem GNPT in einem Reaktionsgefäß durchgeführt wird.

12. Verfahren nach einem der vorstehenden Ansprüche, wobei die Bedingungen, die eine Phosphorylierung ermöglichen, ferner umfassen:
i) Inkubieren des mannosereichen Naglu-Proteins mit dem GNPT in Gegenwart von UDP-GlcNac; und/oder
ii) Inkubieren des mannosereichen Naglu-Proteins mit dem GNPT bei etwa 20 °C.

13. Verfahren nach einem der vorstehenden Ansprüche, wobei das Verfahren ferner einen Schritt des Inkubierens des M6P-haltigen Naglu mit N-Acetylglucosamin-1-Phosphodiester-α-N-acetylglucosa minidase umfasst.

14. Verfahren nach einem der vorstehenden Ansprüche, wobei das Naglu-Protein eine Aminosäuresequenz der SEQ ID NO: 1 umfasst.

15. Verfahren nach einem der vorstehenden Ansprüche, wobei das Naglu-Protein nicht mit einem Peptid fusioniert oder konjugiert ist.

## Revendications

1. Procédé de production d'alpha-N-acétyl-glucosaminidase (Naglu) contenant du mannose-6-phosphate (M6P), comprenant les étapes de :
mise à disposition d'une protéine Naglu contenant un taux élevé de mannose qui a été générée à partir de cellules mammifères ou non mammifères à l'aide de technologie d'ADN recombinant ; et
mise en contact de la protéine Naglu contenant un taux élevé de mannose avec de la N-acétylglucosamine-1-phosphotransférase (GNPT) dans des conditions qui permettent la phosphorylation d'un ou plusieurs résidus mannose sur la protéine Naglu, les conditions qui permettent la phosphorylation comprenant un rapport molaire de GNPT à Naglu supérieur à 1:1, de sorte que la GNPT est en excès par rapport à la Naglu, générant ainsi une Naglu contenant du M6P.

2. Procédé selon la revendication 1, la protéine Naglu contenant un taux élevé de mannose comprenant des oligosaccharides N-liés Man9(GlcNAc)2, Man8(GlcNAc)2, Man7(GlcNAc)2, Man6(GlcNAc)2, et/ou Man5(GlcNAc)2.

3. Procédé selon la revendication 1 ou 2, la protéine Naglu contenant un taux élevé de mannose comprenant des oligosaccharides N-liés Man9(GlcNAc)2.

4. Procédé selon l'une quelconque des revendications précédentes, la protéine Naglu contenant un taux élevé de mannose étant produite à partir de cellules cultivées en présence d'un inhibiteur d'alpha-mannosidase I.

5. Procédé selon la revendication 4, l'inhibiteur d'alpha-mannosidase I étant choisi dans le groupe constitué par la désoxymannojirimycine, la kifunensine, la D-mannonolactame amidrazone, la N-butyl-désoxymannojirimycine, et des combinaisons correspondantes.

6. Procédé selon la revendication 4 ou 5, l'inhibiteur d'alpha-mannosidase I étant la kifunensine.

7. Procédé selon l'une quelconque des revendications 4 à 6, les cellules étant des cellules mammifères.

8. Procédé selon l'une quelconque des revendications 4 à 7, les cellules étant des cellules humaines ou des cellules CHO.

9. Procédé selon l'une quelconque des revendications 4 à 8, le procédé comprenant en outre une étape de purification de la protéine Naglu contenant un taux élevé de mannose à partir des cellules.

10. Procédé selon l'une quelconque des revendications précédentes, la mise en contact de la protéine Naglu contenant un taux élevé de mannose avec la GNPT étant mise en œuvre *in vitro.*

11. Procédé selon l'une quelconque des revendications précédentes, la mise en contact de la protéine Naglu contenant un taux élevé de mannose avec la GNPT étant mise en œuvre dans une cuve de réaction.

12. Procédé selon l'une quelconque des revendications précédentes, les conditions qui permettent la phosphorylation comprenant en outre :
i) l'incubation de la protéine Naglu contenant un taux élevé de mannose avec la GNPT en présence de UDP-GlcNac ; et/ou
ii) l'incubation de la protéine Naglu contenant un taux élevé de mannose avec la GNPT à environ 20 °C.

13. Procédé selon l'une quelconque des revendications précédentes, le procédé comprenant en outre une étape d'incubation de la Naglu contenant du M6P avec de la N-acétylglucosamine 1-phosphodiester α-N-acétylglucosaminidase.

14. Procédé selon l'une quelconque des revendications précédentes, la protéine Naglu comprenant une séquence d'acides aminés de SED ID NO:1.

15. Procédé selon l'une quelconque des revendications précédentes, la protéine Naglu n'étant pas condensée ou conjuguée à un peptide.
